# EUROPÄISCHE PATENTSCHRIFT

(11) **EP 2 593 158 B1**
(45) Veröffentlichungstag und Bekanntmachung des Hinweises auf die Patenterteilung: **22.04.2015**
(21) Anmeldenummer: 11730970.8
(22) Anmeldetag: 11.07.2011
(51) Int. Cl.: A61M 5/158

(54) **MEDIZINISCHE VORRICHTUNG MIT MEHRTEILIGEM GEHÄUSE**
MEDICAL DEVICE COMPRISING A MULTIPART HOUSING
DISPOSITIF MÉDICAL À BOÎTIER MULTIPLE

(30) Priorität: 12.07.2010 EP 10169259
(43) Veröffentlichungstag der Anmeldung: 22.05.2013
(73) Patentinhaber: F.Hoffmann-La Roche AG, 4070 Basel (CH); Roche Diagnostics GmbH, 68305 Mannheim (DE)
(72) Erfinder: DECK, Frank, 67150 Niederkirchen (DE)
(74) Vertreter: Stößel, Matthias
(86) Internationale Anmeldenummer: PCT/EP2011/061781
(87) Internationale Veröffentlichungsnummer: WO 2012/007437

(56) Entgegenhaltungen:
- EP-A1- 2 077 128
- WO-A1-2006/077262
- DE-U1- 29 905 066
- US-A1- 2001 008 963
- US-A1- 2009 048 563

## Beschreibung

### Gebiet der Erfindung

Die Erfindung betrifft eine medizinische Vorrichtung zur Ausübung mindestens einer medizinischen Funktion, umfassend ein mehrteiliges Gehäuse sowie mindestens ein in ein Körpergewebe eines Benutzers insertierbares Element. Weiterhin betrifft die Erfindung ein Schutzbauteil zum Einsatz in einer medizinischen Vorrichtung sowie ein Verfahren zum Schutz eines Funktionsbauteils einer medizinischen Vorrichtung. Derartige Vorrichtungen und Verfahren werden insbesondere im Bereich der medizinischen Diagnostik eingesetzt, vorzugsweise im Bereich des so genannten Home-Monitorings, insbesondere um eine Konzentration mindestens eines Analyten in einer Körperflüssigkeit, beispielsweise Blut oder interstitielle Flüssigkeit, zu überwachen, beispielsweise eine Glukosekonzentration und/oder Cholesterinkonzentration. Alternativ oder zusätzlich sind jedoch auch andere Anwendungen möglich, wie beispielsweise Anwendungen im Bereich der medizinischen Medikation, beispielsweise zur Verabreichung von Medikamenten wie Insulin, insbesondere im Rahmen von Insulinpumpen.

### Stand der Technik

Aus dem Bereich der medizinischen Diagnostik oder Therapeutik sind subkutane Vorrichtungen bekannt, also Vorrichtungen, welche eingerichtet sind, um vollständig oder teilweise in ein Körpergewebe, beispielsweise ein interstitielles Fettgewebe, eingesetzt zu werden. Siehe beispielsweise US 2009/0048563. Dieses Einsetzen wird in der Regel auch als Insertion oder Implantation bezeichnet. Beispiele derartiger subkutaner Vorrichtungen sind insbesondere im Bereich der Diagnostik zu finden, beispielsweise im Bereich der Langzeitüberwachung von Probanden bzw. Benutzern, insbesondere im Rahmen des so genannten "Home-Monitorings" oder auch im Klinikbereich. Dementsprechend können medizinische Vorrichtungen mit mindestens einem insertierbaren Element ausgestattet werden, beispielsweise einem insertierbaren Sensor zum qualitativen und/oder quantitativen Nachweis mindestens eines Analyten (beispielsweise mindestens eines Metaboliten) in dem Körpergewebe und/oder einer Körperflüssigkeit. Derartige Sensoren können beispielsweise auf elektrochemischen und/oder optischen Nachweisprinzipien basieren. Alternativ oder zusätzlich kann die medizinische Vorrichtung auch andere Arten insertierbarer Elemente umfassen, wie beispielsweise Medikationsvorrichtungen, welche im Körpergewebe gezielt und vorzugsweise dosiert bestimmte Wirkstoffe einbringen können, beispielsweise Insulin. Ohne Beschränkung weiterer möglicher Einsatzgebiete wird die Erfindung im Nachfolgenden im Wesentlichen unter Bezugnahme auf medizinische Vorrichtungen mit insertierbaren Sensoren und/oder unter Bezugnahme auf Insulinpumpen beschrieben.

In der medizinischen Therapeutik und/oder Diagnostik ist es häufig erforderlich, ein oder mehrere physikalische und/oder chemische Parameter in einem Körpergewebe eines Benutzers zu erfassen. Beispiele derartiger physikalischer und/oder chemischer Parameter sind Analytkonzentrationen eines oder mehrerer Analyte, wie beispielsweise Glukose und/oder Cholesterin. Abhängig von den erfassten Parametern kann beispielsweise eine medizinische Behandlung gewählt werden, beispielsweise eine Verabreichung bestimmter Medikamente und/oder eine anders gestaltete Einflussnahme auf den Körper oder Körperfunktionen des Probanden. Insbesondere zum qualitativen oder quantitativen Nachweis eines oder mehrerer Analyte durch insertierbare, subkutane Sensoren, welche vorzugsweise durch eine Hautoberfläche eines Benutzers hindurch in ein Körpergewebe, beispielsweise ein Fettgewebe ragen, sind aus dem Stand der Technik zahlreiche Beispiele bekannt. So können insertierbare Sensoren beispielsweise auf elektrochemischen Messprinzipien basieren und eine oder mehrere chemische Substanzen, welche auch als Testchemie bezeichnet werden, umfassen, welche eine oder mehrere physikalisch und/oder chemisch messbare Eigenschaften bei Anwesenheit des mindestens einen nachzuweisenden Analyten spezifisch verändern. Beispiele derartiger Testchemien sind auf Enzymen basierende Testchemien, welche beispielsweise in elektrochemischen Sensoren eingesetzt werden können. Andere Messprinzipien basieren beispielsweise auf optischen Eigenschaften, bei denen mindestens eine Testchemie mindestens eine optisch nachweisbare Eigenschaft bei Anwesenheit des mindestens einen nachzuweisenden Analyten ändert. Auf sämtliche bekannte Messprinzipien kann im Rahmen der vorliegenden Erfindung Bezug genommen werden.

Derartige medizinische Vorrichtungen mit insertierbaren Sensoren dienen in der Regel der Langzeitmessung, beispielsweise kontinuierlich oder diskontinuierlich über einen Zeitraum von mehreren Tagen, beispielsweise sieben Tagen, oder auch mehreren Wochen bis hin zu einem oder mehreren Monaten, hinweg. Während dieser Zeit verbleiben die insertierbaren Sensoren zumindest abschnittsweise innerhalb des Körpergewebes. Eine technische und medizinische Herausforderung besteht dabei in der Regel darin, den insertierbaren Sensor möglichst schmerzfrei und zuverlässig in das Körpergewebe, beispielsweise das subkutane Fettgewebe zu insertieren. Die Insertion sollte derart erfolgen, dass der insertierbare Sensor über einen längeren Zeitraum von beispielsweise mehreren Stunden bis hin zu mehreren Tagen zumindest teilweise in dem Körpergewebe verbleiben kann und beispielsweise kontinuierlich oder diskontinuierlich Messdaten liefern kann. Aus dem Stand der Technik sind daher eine Reihe von Insertionsvorrichtungen bekannt, welche neben dem insertierbaren Element, beispielsweise dem Sensor, eine oder mehrere Insertionshilfen umfassen. Derartige Insertionshilfen können beispielsweise ganz oder teilweise als Insertionsnadeln und/oder Kanülen ausgestaltet sein, in welche das insertierbare Element eingebracht oder auf welche die insertierbaren Elemente aufgebracht werden können, um mit diesen in das Körpergewebe implantiert zu werden. Anschließend kann die Insertionshilfe wieder entfernt werden, wobei das insertierbare Element, beispielsweise der Sensor, zumindest teilweise in dem Körpergewebe verbleibt. Ein Teil des insertierbaren Elements kann dabei aus dem Körpergewebe herausragen, beispielsweise für eine spätere Entfernung des insertierbaren Elements und/oder für eine Verbindung mit einer Ansteuerelektronik.

Bekannte medizinische Vorrichtungen in der Form von Langzeitsensoren (continuous monitoring, CM) weisen in der Regel mindestens einen insertierbaren Sensor auf, welcher in das subkutane Fettgewebe appliziert wird, und mindestens einen peripheren Baustein, welcher in der Regel direkt oder indirekt auf eine Hautoberfläche des Benutzers appliziert wird. Das mindestens eine periphere Element weist in der Regel beispielsweise mindestens eine Verstärkungselektronik zur Verstärkung von Sensorsignalen und/oder zur Ansteuerung des Sensors auf. Alternativ oder zusätzlich können weitere Module enthalten sein, beispielsweise ein oder mehrere Funkmodule, ein oder mehrere Energiespeicher und eine oder mehrere Schutzeinrichtungen. Neben diesen Elementen werden zur Applikation des Sensors die oben beschriebenen Insertionsvorrichtungen verwendet, welche auch als "Serter" bezeichnet werden, die in der Regel nur für den Zweck der Insertion zum Einsatz kommen und danach in der Regel von der medizinischen Vorrichtung entfernt werden.

Direkt nach der Insertion des Sensors bzw. des insertierbaren Elements kann es zu Blutungen an der Einstichstelle kommen. Derartige Blutungen stoppen in der Regel nach wenigen Minuten von selbst und haben in der Regel keinen Einfluss auf die Messqualität der medizinischen Vorrichtung. Allerdings stellen sie unter Umständen ein hygienisches Problem dar, sofern das ausgetretene Blut nicht entfernt wird und dieses während der Tragedauer der medizinischen Vorrichtung auf der Wunde verbleibt. Weiterhin werden zur Halterung der peripheren Elemente auf der Hautoberfläche in der Regel Pflaster verwendet, deren Klebekraft durch das benetzende Blut stark eingeschränkt werden kann.

Weiterhin ist aus klinischen Studien bekannt, dass insertierte Langzeitsensoren durch äußere mechanische Einflüsse wie Druck und Zug in ihrer messtechnischen Leistungsfähigkeit und in ihren Signalqualitäten beeinflusst werden. Weiterhin ist bekannt, dass eine dauerhafte Relativbewegung zwischen dem Sensorschaft, welcher durch die Hautoberfläche ragt, und der Wundöffnung zur Reizung der Wundränder und so zu einer Rhagadenbildung führt. Die daraus resultierende Wundreizung und mögliche Entzündungen haben ebenfalls einen negativen Einfluss auf die Leistungsfähigkeit und die Eigenschaften des Sensors.

Denkbare konstruktive Maßnahmen zur Verhinderung von Relativbewegungen zwischen dem Sensorschaft und der Wundöffnung sowie die Reduzierung mechanischer Einflüsse auf das umliegende Gewebe haben jedoch konstruktive Probleme zur Folge oder weisen konstruktiv einige Herausforderungen auf. So würde beispielsweise ein einfacher mechanischer Schutz, wie er zur Behebung der aufgeführten Probleme eingesetzt werden könnte, zu einem räumlichen Problem führen. Ein mechanischer Schutz zur Verhinderung von Relativbewegungen zwischen Sensorschaft und Wundöffnung müsste das Gewebe, welches sich um die Wunde herum befindet, stabilisieren und mechanisch mit der Sensorbasis, also dem periphären Bauelement, beispielsweise dem Funktionsbauteil, auf der Hautoberfläche verbinden. Dazu sind möglichst geringe Abstände zwischen Sensorbasis und Wunde von Vorteil. Geringe Abstände bringen aber erhebliche Hygieneprobleme mit sich, da kurz nach der Insertion austretendes Blut eine dicht benachbarte Sensorbasis sehr schnell berührt und sich aufgrund von Kapillarkräften zwischen Sensorbasis und Haut zieht. Eine Reinigung von Tupfern ist durch die mechanische Stabilisierung aus Platzgründen ebenfalls erschwert. Weiterhin wäre zur Reduzierung mechanischer Einflüsse auf das Gewebe, in welches der Sensor insertiert ist, die Stabilisierung und Abdeckung des gesamten Gewebeareals notwendig. Dazu müsste der mechanische Schutz jedoch eine Größe besitzen, die mindestens der Länge des insertierten Sensors entspricht. Ein mechanischer Schutz dieser Größe ist aber sehr hinderlich bei der Insertion des Sensors, da Insertionshilfen, beispielsweise der oben beschriebenen Art, selbst eine räumliche Ausdehnung aufweisen, welche mit den Abmessungen denkbarer Schutzvorrichtungen kollidieren würde.

### Aufgabe der Erfindung

Es ist dementsprechend eine Aufgabe der Erfindung, eine medizinische Vorrichtung mit mindestens einem insertierbaren Element bereitzustellen, welche die Nachteile bekannter medizinischer Vorrichtungen vermeidet. Insbesondere soll ein mechanischer Schutz des insertierten Elements gewährleistet werden. Gleichzeitig soll eine Insertionsstelle jedoch auf einfache Weise für eine Reinigung zugänglich sein, und eine Insertion des insertierbaren Elements in ein Körpergewebe soll trotz des mechanischen Schutzes auf einfache Weise möglich sein.

### Offenbarung der Erfindung

Diese Aufgabe wird gelöst durch eine Vorrichtung und ein Verfahren mit den Merkmalen der unabhängigen Ansprüche. Vorteilhafte Weiterbildungen der Erfindung, welche einzeln oder in Kombination realisierbar sind, sind in den abhängigen Ansprüchen dargestellt.

In einem ersten Aspekt der vorliegenden Erfindung wird eine medizinische Vorrichtung zur Ausübung mindestens einer medizinischen Funktion vorgeschlagen. Unter einer medizinischen Funktion ist dabei allgemein eine diagnostische und/oder therapeutische und/oder chirurgische Funktion zu verstehen. Insbesondere kann es sich um eine diagnostische Funktion handeln, also eine Funktion zur Erkennung mindestens eines Körperzustands eines Benutzers, beispielsweise eine sensorische Funktion, insbesondere eine Sensorfunktion zum Nachweis mindestens eines Analyten in einer Körperflüssigkeit. Alternativ oder zusätzlich kann die medizinische Funktion jedoch auch beispielsweise eine therapeutische Funktion umfassen, also eine Funktion, welche mindestens eine heilende Wirkung und/oder regulative Wirkung auf den Körper ausübt und/oder welche mindestens einen Einfluss auf mindestens einen Körperzustand ausüben kann. Insbesondere kann es sich hierbei um eine Medikationsfunktion handeln, also eine Funktion, welche mindestens ein Medikament an den Körper verabreicht, beispielsweise gesteuert und/oder geregelt, beispielsweise Insulin oder ein anderes Medikament. Auch Kombinationen der genannten medizinischen Funktionen sind möglich, beispielsweise indem die medizinische Vorrichtung gleichzeitig eine diagnostische Funktion, beispielsweise in Form einer Messung einer Glukosekonzentration, und mindestens eine Medikationsfunktion, beispielsweise in Form einer Insulinmedikation, beispielsweise in Form einer Insulinpumpe, in ein und derselben medizinischen Vorrichtung integriert, beispielsweise innerhalb desselben Gehäuses. Die medizinische Vorrichtung kann insbesondere im zusammengesetzten Zustand als einzelnes Bauteil ausgestaltet sein und kann insbesondere eingerichtet sein, um am Körper des Benutzers getragen zu werden, beispielsweise auf einer Hautoberfläche, beispielsweise im Bauchbereich, im Armbereich oder im Rückenbereich des Benutzers. Zu diesem Zweck kann die medizinische Vorrichtung beispielsweise insgesamt ein Volumen von vorzugsweise nicht mehr als 200 ccm, insbesondere von nicht mehr als 100 ccm und besonders bevorzugt von nicht mehr als 50 ccm oder sogar nicht mehr als 10 ccm aufweisen. Die medizinische Vorrichtung kann mit weiteren Geräten zusammenwirken, beispielsweise einem externen, nicht drahtgebunden mit der medizinischen Vorrichtung verbundenen Datenmanager und/oder einem Steuergerät. Alternativ oder zusätzlich kann die Anbindung auch beispielsweise eine fluidische Anbindung umfassen, beispielsweise eine Schlauchanbindung an eine Medikationspumpe wie z.B. eine Insulinpumpe. Verschiedene Ausgestaltungen sind möglich.

Die medizinische Vorrichtung umfasst mindestens ein zumindest teilweise in ein Körpergewebe eines Benutzers insertierbares Element. Dieses insertierbare Element kann auf die mindestens eine medizinische Funktion angepasst sein. Wie oben dargestellt, ist dabei unter einer Insertion eine vollständige oder teilweise Einbringung in ein Körpergewebe zu verstehen. Diese Insertion erfolgt vorzugsweise derart, dass ein Teil des insertierbaren Elements durch eine Hautoberfläche des Benutzers hindurch in das Körpergewebe geführt ist. Das insertierbare Element kann beispielsweise, wie unten noch näher ausgeführt wird, mindestens einen Sensor zum Nachweis mindestens eines Analyten in einer Körperflüssigkeit umfassen, insbesondere einen elektrochemischen und/oder einen optischen Sensor. Alternativ oder zusätzlich kann das insertierbare Element auch ein insertierbares Teil einer Medikationsvorrichtung umfassen, beispielsweise eine insertierbare Kanüle, welche mit anderen Teilen der medizinischen Vorrichtung zusammenwirken kann, beispielsweise einer Medikationspumpe, insbesondere einer Insulinpumpe. Auch Kombinationen sind möglich, beispielsweise eine Kombination mindestens eines insertierbaren Sensors mit mindestens einer Medikationsvorrichtung.

Weiterhin umfasst die medizinische Vorrichtung mindestens ein auf einer Hautoberfläche des Benutzers aufbringbares Gehäuse. Das Gehäuse kann beispielsweise Bestandteil einer Basis der medizinischen Vorrichtung sein oder kann die Basis der medizinischen Vorrichtung bilden, wobei die medizinische Vorrichtung somit das insertierbare Element und die Basis umfasst oder vollständig aus dem insertierbaren Element und der Basis besteht. Das insertierbare Element kann, wie unten noch näher ausgeführt wird, mit der Basis verbindbar sein oder sogar fest mit der Basis verbunden sein, so dass die medizinische Vorrichtung vorzugsweise im zusammengesetzten Zustand einstückig ausgebildet ist.

Erfindungsgemäß wird vorgeschlagen, das Gehäuse mehrteilig auszugestalten. So umfasst das Gehäuse mindestens ein Funktionsbauteil, welches mit dem insertierbaren Element verbindbar ist. Unter einem Funktionsbauteil ist dabei grundsätzlich ein beliebiges Bauteil der medizinischen Vorrichtung zu verstehen, welches mit dem insertierbaren Element verbunden werden kann und beispielsweise mit diesem im verbundenen Zustand mechanisch und/oder elektrisch und/oder fluidisch zusammenwirken kann. Das Funktionsbauteil kann beispielsweise, wie unten noch näher ausgeführt wird, mindestens eine elektronische und/oder elektromechanische und/oder fluidische Komponente umfassen, wie beispielsweise einen oder mehrere elektronische Bausteine. Beispielsweise kann eine Ansteuer- und Auswerteschaltung des Sensors ganz oder teilweise in dem Funktionsbauteil umfasst sein. Insbesondere kann das Funktionsbauteil mindestens eine Verstärkerschaltung und/oder mindestens einen Potentiostaten für einen elektrochemischen Sensor umfassen. Alternativ oder zusätzlich können weitere Elemente in dem Funktionsbauteil umfasst sein, wie beispielsweise ein oder mehrere Energiespeicher, ein oder mehrere Speicherbauelemente, ein oder mehrere Kommunikationsbausteine, wie beispielsweise Funkchips oder Kombinationen der genannten und/oder anderer Elemente. Insbesondere kann das Funktionsbauteil mit den darin enthaltenen Komponenten, gemeinsam mit dem insertierbaren Element, unabhängig von weiteren Komponenten der mechanischen Vorrichtungen funktionieren bzw. mindestens eine unabhängig von weiteren Komponenten der mechanischen Vorrichtung, insbesondere von dem Gehäuse der mechanischen Vorrichtung, ausübbare Funktion umfassen. So können beispielsweise eine Sensorfunktion und/oder eine Medikationsfunktion allein durch das insertierbare Element und das Funktionsbauteil mit den darin umschlossenen Elementen ausübbar sein.

Das Funktionsbauteil kann einteilig ausgebildet sein. Alternativ kann das Funktionsbauteil jedoch seinerseits mehrteilig ausgestaltet sein und beispielsweise zwei oder mehr miteinander verbindbare Funktionsbauteil-Komponenten umfassen. So kann beispielsweise eine erste Funktionsbauteil-Komponente vorgesehen sein, welche mit dem insrtierbaren Element verbindbar oder verbunden ist, und eine zweite Funktionsbauteil-Komponente, welche wiederum direkt oder indirekt mit der ersten Funktionsbauteil-Komponente verbindbar ist, beispielsweise vor, während oder nach der Insertion. So kann beispielsweise das Funktionsbauteil selbst in einen Einweg-Teil (Disposable) mit dem insertierbaren Element und einen wiederverwendbaren Teil (Reusable) untergliedert sein. In dem wiederverwendbaren Teil können beispielsweise eine oder mehrere wiederverwendbare Elektronikkomponenten und/oder elektromechanische Komponenten und/oder fluidische Komponenten aufgenommen sein, beispielsweise eine, mehrere oder alle der oben beschriebenen Komponenten. In dem Disposable kann beispielsweise das insertierbare Element fest enthalten sein, oder das insertierbare Element kann mit dem Disposable verbindbar sein, beispielsweise durch einen in dem Disposable enthaltenen Stecker oder Verbinder. Weiterhin kann in dem Disposable mindestens eine Elektronikkomponente und/oder elektromechanische Komponente und/oder fluidische Komponente enthalten sein. Eine Verbindung zwischen den Funktionsbauteil-Komponenten kann beispielsweise eine lösbare Verbindung umfassen, beispielsweise eine Steckverbindung. Die Verbindung kann beispielsweise eine mechanische und/oder elektrische und/oder fluidische Verbindung zwischen den Funktionsbauteü-Komponenten beinhalten oder herstellen.

Die Verbindung zwischen dem insertierbaren Element und dem Funktionsbauteil kann fest oder lösbar ausgestaltet sein. Vorzugsweise handelt es sich um eine physikalische Verbindung fester Natur. Beispielsweise kann das insertierbare Element in das Funktionsbauteil hineingeführt sein und dort fest mit diesem verbunden sein. Grundsätzlich ist jedoch auch eine andere Art der Verbindung möglich, sogar prinzipiell eine drahtlose Verbindung. Die Integration der optionalen mindestens einen Komponente, welche von dem Funktionsbauteil umschlossen wird, in das Funktionsbauteil kann auf verschiedene Weisen erfolgen. So kann die mindestens eine Komponente, beispielsweise das mindestens eine elektrische und/oder elektronische und/oder elektromechanische und/oder fluidische Bauteil, in ein Inneres des Funktionsbauteils eingebracht sein und von dem Funktionsbauteil beispielsweise umschlossen sein. Alternativ oder zusätzlich kann die mindestens eine Komponente jedoch auch selbst Bestandteil des Funktionsbauteils sein, beispielsweise indem dieses in das Funktionsbauteil integriert ist. So kann beispielsweise die Wand eines elektronischen Chipgehäuses in das Funktionsbauteil integriert sein, beispielsweise indem dieses umspritzt oder auf andere Weise von einem Werkstoff des Funktionsbauteils umschlossen wird und somit beispielsweise selbst Bestandteil eines Wandmaterials des Funktionsbauteils ist. Verschiedene Ausgestaltungen sind denkbar.

Wie oben ausgeführt, ist erfindungsgemäß vorgesehen, dass das Gehäuse mehrteilig ausgestaltet ist. Dies bedeutet, dass das Gehäuse neben dem mindestens einem Funktionsbauteil ein oder mehrere weitere Elemente umfasst, welche zusammen das Gehäuse bilden. Dementsprechend wird vorgeschlagen, dass das Gehäuse weiterhin mindestens ein Schutzbauteil aufweist. Unter einem Schutzbauteil ist dabei ein Bauteil des Gehäuses zu verstehen, welches einen Schutz für das Funktionsbauteil und/oder das insertierbare Element bereitstellt, insbesondere einen mechanischen Schutz, beispielsweise gegen Druckbelastungen und/oder Zugbelastungen und/oder Biegebelastungen. Insbesondere kann ein Schutz gegenüber einer Druck-, Zug- oder Biegebelastung des in das Körpergewebe insertierten Elements bereitgestellt werden.

Das Schutzbauteil ist eingerichtet, um das Funktionsbauteil zumindest teilweise zu umschließen. Unter einer Umschließung wird dabei, wie unten noch näher ausgeführt, verstanden, dass das Schutzbauteil eingerichtet ist, um in mindestens einer Dimension, beispielsweise in mindestens einer Ebene, beispielsweise parallel zu einer Hautoberfläche des Benutzers, das Funktionsbauteil vollständig oder zumindest über einen Winkelbereich hinweg zu umfassen oder zu umranden, wobei die Umfassung vollständig oder teilweise erfolgen kann. Mögliche Ausgestaltungen werden unten noch mehr beschrieben.

Das Schutzbauteil ist dabei eingerichtet, um mit dem Funktionsbauteil verbunden zu werden. Diese Verbindbarkeit kann insbesondere derart ausgestaltet sein, dass diese auch nach einer Insertion des insertierbaren Elements erfolgen kann. So kann zunächst eine Insertion des insertierbaren Elements erfolgen, gefolgt von einer Verbindung des Schutzbauteils mit dem Funktionsbauteil. Unter einer Verbindung zwischen dem Schutzbauteil und dem Funktionsbauteil wird dabei die Zusammenfügung dieser Bauteile zu einem gemeinsamen Bauteil verstanden. Diese Verbindung kann direkt oder auch indirekt erfolgen, so dass sich in verbundenem Zustand das Funktionsbauteil und das Schutzbauteil berühren können. Auch eine berührungslose Verbindung ist jedoch möglich, beispielsweise indem zwischen dem Funktionsbauteil und dem Schutzbauteil mindestens ein Spalt entsteht und/oder indem die Verbindung über mindestens ein Zwischenelement erfolgt. Als Zwischenelement können dabei beispielsweise ein gemeinsames Pflaster und/oder auch die Haut des Benutzers wirken, beispielsweise in Form einer Brücke. Das Funktionsbauteil und/oder das Schutzbauteil können derart ausgestaltet sein, dass diese Verbindbarkeit gewährleistet sein kann. Beispielsweise können ein oder mehrere Verbindungselemente jeweils an dem Funktionsbauteil und/oder dem Schutzbauteil vorgesehen sein. Beispielsweise kann auch eine äußere Form des Funktionsbauteils derart ausgestaltet sein, dass diese zumindest teilweise in eine Aufnahme des Schutzbauteils eingefügt werden kann. Unter "verbindbar" ist also im Rahmen der vorliegenden Erfindung zu verstehen, dass das Funktionsbauteil und das Schutzbauteil kombiniert werden können, wobei beim Verbinden die Vorrichtung oder ein Teil derselben entsteht, wobei in dem verbundenen Zustand das Funktionsbauteil und das Schutzbauteil in einer vorgegebenen Ausrichtung und/oder Anordnung zueinander angeordnet sind, so dass das Schutzbauteil seine Schutzfunktion entfalten kann.

Dieses gemeinsame Bauteil kann beispielsweise eine Basis der medizinischen Vorrichtung sein. Beispielsweise kann diese Basis vollständig oder teilweise direkt oder indirekt auf eine Hautoberfläche des Benutzers aufgebracht werden. Beispielsweise kann eine äußere Form des Gehäuses im verbundenen Zustand zumindest in einer Ebene, beispielsweise einer Ebene parallel zur Hautoberfläche, durch das Schutzbauteil definiert sein, so dass das Schutzbauteil die äußeren Abmessungen des Gehäuses in zumindest dieser Schnittebene definieren kann. Vorzugsweise, jedoch nicht notwendigerweise, berührt das Schutzbauteil das Funktionsbauteil im verbundenen Zustand. Die Verbindung zwischen dem Funktionsbauteil und dem Schutzbauteil kann direkt erfolgen oder kann auch, alternativ oder zusätzlich, durch andere Elemente gegeben sein, beispielsweise durch ein oder mehrere Verbindungselemente, wie unten exemplarisch näher ausgeführt wird.

Die medizinische Vorrichtung kann vorzugsweise derart ausgestaltet sein, dass das Schutzbauteil das Funktionsbauteil derart umschließt, dass mindestens eine Insertionsstelle, an welcher das insertierbare Element die Hautoberfläche des Benutzers durchdringt, zugänglich ist. Diese Zugänglichkeit kann beispielsweise einen Zugriff von oben, von einer der Hautoberfläche gegenüberliegenden Seite der Basis bzw. des Gehäuses ermöglichen, beispielsweise einen Zugriff mittels eines Wattestäbchens oder einer anderen Reinigungsvorrichtung, beispielsweise zum Reinigen und/oder zum Desinfizieren der Insertionsstelle und/oder zum Zweck einer Beobachtung der Insertionsstelle. Beispielsweise kann um die Insertionsstelle bei mit dem Funktionsbauteil verbundenem Schutzbauteil ein Freiraum um die Insertionsstelle herum verbleiben, beispielsweise ein Freiraum, durch welchen hindurch ein Benutzer von einer Außenseite her durch das Schutzbauteil und das Funktionsbauteil hindurch die Insertionsstelle beobachten bzw. diese reinigen und/oder auf andere Weise behandeln kann. Allgemein kann die Vorrichtung derart ausgestaltet sein, dass die Insertionsstelle optisch zugänglich ist. Beispielsweise kann dieser Freiraum eine laterale Abmessung, beispielsweise einen Durchmesser (beispielsweise einen Kreisdurchmesser und/oder eine andere, eine laterale Ausdehnung charakterisierende Größe) und/oder eine Kantenlänge, von mindestens 2 mm, vorzugsweise mindestens 3 mm und besonders bevorzugt mindestens 5 mm oder sogar mindestens 7 mm oder sogar mindestens 10 mm umfassen. Beispielsweise kann ein Freiraum von 2 mm bis 20 mm, insbesondere von 3 mm bis 15 mm und besonders bevorzugt von 5 mm bis 15 mm lateraler Ausdehnung vorgesehen sein. Insbesondere kann die Insertionsstelle in einer Richtung parallel zur Hautoberfläche, beispielsweise in der Ebene der Hautoberfläche, vollständig von dem Gehäuse umgeben sein, beispielsweise ringförmig oder rahmenförmig. Der Freiraum kann dabei grundsätzlich einen beliebigen Querschnitt aufweisen, beispielsweise einen ovalen, runden, polygonalen oder auf andere Weise gestalteten Querschnitt.

Um eine Stabilisierung der medizinischen Vorrichtung bei in das Körpergewebe insertiertem insertierbaren Element besonders wirkungsvoll bereitstellen zu können und beispielsweise eine Druckbelastung und/oder Zugbelastung und/oder Wiegebelastung des insertierten Elements zu verringern, ist es besonders bevorzugt, wenn die Insertionsstelle im Wesentlichen mittig zu einer Auflagefläche des Gehäuses auf der Hautoberfläche angeordnet ist. Unter im Wesentlichen mittig ist dabei zu verstehen, dass die Insertionsstelle, also ein Punkt oder ein Bereich, in welchem bei bestimmungsgemäßer Verwendung der medizinischen Vorrichtung ein Durchbruch des insertierbaren Elements durch die Hautoberfläche erfolgt, im Wesentlichen im Flächenmittelpunkt der Auflagefläche angeordnet ist. Beispielsweise kann die Insertionsstelle im Flächenmittelpunkt der Projektion des optionalen Freiraums auf die Hautoberfläche angeordnet sein, oder der Flächenmittelpunkt des optionalen Freiraums auf die Hautoberfläche kann im Wesentlichen im Flächenmittelpunkt der Auflagefläche angeordnet sein. Unter einer Anordnung "im Wesentlichen im Flächenmittelpunkt" ist dabei allgemein zu verstehen, dass die Abweichung der Punkte vom Flächenmittelpunkt in keiner Richtung mehr als 50 % der maximal lateralen Ausdehnung (beispielsweise eines Durchmessers) der Auflagefläche beträgt, vorzugsweise nicht mehr als 20 %, besonders bevorzugt nicht mehr als 10 % und insbesondere nicht mehr als 5 %.

Wie oben dargestellt, kann das insertierbare Element insbesondere einen Sensor zum Nachweis mindestens eines Analyten in einer Körperflüssigkeit umfassen. Insbesondere kann es sich hierbei um einen elektrochemischen Sensor und/oder einen optischen Sensor handeln, beispielsweise einen Sensor zum qualitativen und/oder quantitativen Nachweis von Blutglukose und/oder Cholesterin. Alternativ oder zusätzlich kann das insertierbare Element jedoch auch mindestens einen Bestandteil einer Medikationsvorrichtung umfassen. Beispielsweise mindestens einen Bestandteil einer Insulinpumpe, beispielsweise mindestens eine Kanüle. Diese Kanüle kann beispielsweise flexibel oder auch starr ausgestaltet sein und kann beispielsweise einen Schlauchanschluss umfassen, um mittels eines flexiblen Schlauchs mit einer Pumpe, beispielsweise einer Medikationspumpe und insbesondere einer Insulinpumpe, verbunden zu werden.

Wie oben ausgeführt, kann das Schutzbauteil insbesondere reversibel mit dem Funktionsbauteil verbindbar sein. Dementsprechend kann das Funktionsbauteil mit dem insertierbaren Element beispielsweise als über einen längeren Zeitraum verwendbares Element ausgestaltet sein, wohingegen das Schutzbauteil in regelmäßigen oder unregelmäßigen Abständen ausgetauscht werden kann, beispielsweise um eine Hygiene der gesamten medizinischen Vorrichtung zu erhöhen. Das Schutzbauteil kann das Funktionsbauteil insbesondere in Form eines Rahmens umschließen. Beispielsweise kann in einer Draufsicht mit Blickrichtung senkrecht zur Hautoberfläche in montiertem Zustand, also bei mit dem Funktionsbauteil verbundenem Schutzbauteil, sowohl das Funktionsbauteil als auch das Schutzbauteil erkennbar sein und vorzugsweise auch eine Insertionsstelle. Dies bedeutet, dass vorzugsweise das Schutzbauteil das Funktionsbauteil lediglich in lateraler Richtung, also in einer Ebene parallel zur Hautoberfläche, umschließt, wobei eine Umschließung senkrecht zu dieser Ebene, also in einer Richtung senkrecht zur Hautoberfläche, zwar grundsätzlich ebenfalls möglich ist, jedoch nicht bevorzugt ist. Das Funktionsbauteil und das Schutzbauteil weisen vorzugsweise in einer Richtung senkrecht zur Hautoberfläche im Wesentlichen dieselbe Höhe auf. Beispielsweise können die maximalen Höhen dieser Bauteile voneinander um nicht mehr als 50 % abweichen. Auch andere Ausgestaltungen sind jedoch grundsätzlich möglich.

Besonders bevorzugt ist es, wenn das Funktionsbauteil eine Längsseite aufweist, wobei das Schutzbauteil das Funktionsbauteil zumindest entlang der gesamten Längsseite umschließt. Besonders bevorzugt ist es jedoch, wenn das Schutzbauteil über die Längsseite des Funktionsbauteils hinausragt, beispielsweise um auch eine Projektion des insertierbaren Elements, beispielsweise des Sensors, auf die Hautoberfläche mit zu umschließen. Allgemein kann beispielsweise ein äußerer Rand des Schutzbauteils eine Projektion des Funktionsbauteils und des insertierbaren Elements auf die Hautoberfläche des Benutzers vollständig umschließen. Dies bedeutet, dass auch im insertierten Zustand des insertierbaren Elements die Projektion des insertierbaren Elements auf die Hautoberfläche des Benutzers vollständig vom Gehäuse erfasst wird, also entweder von dem Funktionsbauteil oder von dem Schutzbauteil.

Wie oben dargestellt, weist das Funktionsbauteil vorzugsweise mindestens eine Funktion auf, beispielsweise die Funktion einer Aufnahme und/oder Auswertung von Signalen des Sensors und/oder eine fluidische Funktion, beispielsweise eine Pumpenfunktion und/oder eine andere elektromechanische und/oder elektrische und/oder fluidische Funktion. Besonders bevorzugt ist es, wenn mindestens eine Funktion des Funktionsbauteils unabhängig von dem Vorhandensein des Schutzbauteils ausübbar ist. Insbesondere kann es sich hierbei um mindestens eine medizinische Funktion handeln, welche von dem Funktionsbauteil in Zusammenwirkung mit dem insertierbaren Element ausübbar ist, vorzugsweise im insertierten Zustand. Beispielsweise kann es sich hierbei um eine Nachweisfunktion zum Nachweis mindestens eines Analyten in der Körperflüssigkeit handeln und/oder eine Medikationsfunktion. Dies bedeutet, dass in diesem Zusammenhang das Schutzbauteil beliebig von dem Funktionsbauteil entfernbar ist, ohne die mindestens eine medizinische Funktion zu stören, oder dass die medizinische Funktion bereits ausübbar ist, bevor das Schutzbauteil mit dem Funktionsbauteil verbunden ist. Alternativ oder zusätzlich kann das Funktionsbauteil jedoch, wie unten ausgeführt, ebenfalls mindestens eine Funktion aufweisen, beispielsweise eine unabhängige Funktion. Alternativ oder zusätzlich kann das Schutzbauteil auch mindestens eine Funktion aufweisen, welche in Zusammenwirkung mit dem Funktionsbauteil ausgeübt wird. Verschiedene Ausgestaltungen sind möglich und werden unten exemplarisch noch näher erläutert.

Wie oben ausgeführt, ist das Schutzbauteil mit dem Funktionsbauteil verbindbar. Diese Verbindung kann auf verschiedene Weisen erfolgen. Insbesondere kann das Schutzbauteil eingerichtet sein, um formschlüssig und/oder kraftschlüssig mit dem Funktionsbauteil verbunden zu werden. Beispielsweise kann ein Formschluss und/oder Kraftschluss durch ein einfaches Umschließen erfolgen, wobei das Schutzbauteil beispielsweise mindestens eine an das Funktionsbauteil angepasste Öffnung umfasst, die beispielsweise in mindestens einer Dimension an eine äußere Abmessung des Funktionsbauteils angepasst sein kann, so dass ein einfaches Umschließen erfolgen kann. Gegebenenfalls kann die Öffnung auch geringfügig kleiner dimensioniert sein und flexibel und/oder verformbar ausgestaltet sein, so dass beim Umschließen ein Formschluss und/oder Kraftschluss erfolgt. Alternativ oder zusätzlich zu einem Formschluss und/oder Kraftschluss sind jedoch auch andere Verbindungsarten möglich, beispielsweise Verbindungen durch einen Stoffschluss.

Wie oben ausgeführt, kann das Schutzbauteil insbesondere mindestens eine Öffnung aufweisen, in welche das Funktionsbauteil vollständig oder teilweise eingefügt werden kann. Insbesondere kann es sich hierbei um eine durchgängige Öffnung handeln, also eine Öffnung, welche von einer der Hautoberfläche zuweisenden Seite zu einer der Hautoberfläche abgewandten Seite des Schutzbauteils durchgängig ausgestaltet ist. Insbesondere ist es bevorzugt, wenn eine Form der Öffnung zumindest abschnittsweise einer äußeren Form des Funktionsbauteils angepasst ist, also beispielsweise exakt der äußeren Form des Funktionsbauteils entspricht, lediglich ein geringfügiges Spiel (beispielsweise ein Spiel von nicht mehr als 1 Millimeter oder 0,5 Millimetern) aufweist oder sogar geringfügig kleiner dimensioniert ist als die äußere Form des Funktionsbauteils, wobei im letzteren Fall eine verformbare Ausgestaltung der Wände der Öffnung bevorzugt ist, beispielsweise eine elastische und/oder plastische Verformbarkeit. Insbesondere kann die Öffnung derart ausgestaltet sein, dass bei mit dem Funktionsbauteil verbundenem Schutzbauteil innerhalb der Öffnung um eine Insertionsstelle herum, an welcher das insertierbare Element die Hautoberfläche des Benutzers durchringt, ein von außen zugänglicher Freiraum verbleibt. Beispielsweise kann dieser Freiraum wie oben ausgeführt, eine laterale Abmessung, beispielsweise einen Durchmesser und/oder eine Kantenlänge, von mindestens 2 mm, insbesondere 3 mm und besonders bevorzugt mindestens 5 mm aufweisen. Besonders bevorzugt ist es jedoch, wenn die laterale Abmessung 10 mm nicht übersteigt, vorzugsweise 7 mm nicht übersteigt. Beispielsweise kann die Öffnung, insbesondere die durchgängige Öffnung, in mindestens zwei Bereiche unterteilt sein, beispielsweise in einer Ebene parallel zur Hautoberfläche. So kann mindestens ein erster Bereich vorgesehen sein, in welchem das Funktionsbauteil aufgenommen oder aufnehmbar ist, beispielsweise durch einen Form- oder Kraftschluss, und ein weiterer Bereich, nämlich der Freiraum oder ein den Freiraum umfassender Bereich. Das insertierbare Element kann auf der dem Freiraum zuweisenden Seite des Funktionsbauteils mit dem Funktionsbauteil verbindbar oder verbunden sein. Beispielsweise kann dies eine Stirnseite des Funktionsbauteils sein. Das Funktionsbauteil kann beispielsweise in einer Ebene parallel zur Hautoberfläche eine runde, ovale oder polygonale, beispielsweise rechteckige Form aufweisen. Beispielsweise kann das insertierbare Element auf einer schmalen Stirnseite eines rechteckigen Funktionsbauteils mit dem Funktionsbauteil verbindbar oder verbunden sein, beispielsweise indem das insertierbare Element auf dieser Seite in das Innere des Funktionsbauteils hineingeführt ist. Das Funktionsbauteil kann insbesondere in seiner Form derart asymmetrisch sein und/oder die Öffnung kann derart ausgestaltet sein, dass stets die mit dem insertierbaren Element verbindbare Seite dem Freiraum zuweist, wenn das Funktionsbauteil mit dem Schutzbauteil verbunden ist. Insbesondere kann eine Ausgestaltung der Öffnung und/oder der Form des Funktionsbauteils derart erfolgen, dass das Funktionsbauteil innerhalb der Öffnung nicht drehbar ist.

Das Gehäuse kann insbesondere zumindest teilweise verformbar, vorzugsweise flexibel, ausgestaltet sein. Insbesondere kann das Schutzbauteil aus mindestens einem flexiblen Material hergestellt sein, also insbesondere zumindest bereichsweise flexibel ausgestaltet sein. Das Funktionsbauteil kann grundsätzlich ebenfalls flexibel ausgestaltet sein, ist jedoch vorzugsweise aus einem härteren Werkstoff hergestellt als das Schutzbauteil. Dementsprechend kann das umschließende Schutzbauteil weicher ausgestaltet sein als das zentraler angeordnete Funktionsbauteil, so dass durch das Funktionsbauteil ein höherer mechanischer Schutz für in dem Funktionsbauteil aufgenommene Komponenten gegeben sein kann. Das Schutzbauteil kann beispielsweise eine Shore A-Härte von 20 bis 70 aufweisen, vorzugsweise von 30 bis 50. Das Funktionsbauteil kann insbesondere eine Shore A-Härte von 30 bis 100 aufweisen, vorzugsweise von 40 bis 80. Das Schutzbauteil und das Funktionsbauteil können insbesondere auch mindestens einen biokompatiblen, hautverträglichen Werkstoff umfassen oder ganz oder teilweise aus einem derartigen biokompatiblen, hautverträglichen Werkstoff hergestellt sein. Das Schutzbauteil und/oder das Funktionsbauteil können insbesondere ganz oder teilweise aus einem Silikon und/oder einem Polyurethan hergestellt sein.

Das Funktionsbauteil kann in seiner äußeren Form insbesondere derart bemessen und/oder ausgestaltet sein, dass eine Insertion des insertierbaren Elements bei mit dem Funktionsbauteil verbundenem insertierbaren Element möglich ist. Zu diesem Zweck kann das insertierbare Element beispielsweise an einer Seite des Funktionsbauteils mit dem Funktionsbauteil verbunden sein. Beispielsweise kann es sich hierbei um eine Stirnseite handeln, beispielsweise eine Schmalseite des Funktionsbauteils. Vorzugsweise erfolgt die Verbindung zwischen dem insertierbaren Element und dem Funktionsbauteil somit nicht auf einer der Hautoberfläche zuweisenden Seite des Funktionsbauteils, sondern auf einer anderen Seite des Funktionsbauteils. Beispielsweise kann es sich hierbei um eine Verbindung an einer Fläche handeln, welche im Wesentlichen senkrecht zur Hautoberfläche ausgerichtet ist. Beispielsweise eine Stirnseite des Funktionsbauteils. Wie oben dargestellt, kann die Verbindung fest oder auch lösbar erfolgen. Besonders bevorzugt ist es, wenn das Funktionsbauteil an der Seite, an welcher das insertierbare Element mit dem Funktionsbauteil verbunden oder verbindbar ist, mindestens eine Aussparung umfasst. Beispielsweise kann das Funktionsbauteil mindestens eine dieser Seite zuweisende, schräg zur Hautoberfläche in Richtung dieser Seite verlaufende Vertiefung umfassen, insbesondere eine auf das insertierbare Element zuweisende Nut, zur Erleichterung der Insertion. Beispielsweise kann die Vertiefung, beispielsweise eine Rinne oder Nut, unter einem Winkel von 20° bis 70° zur Hautoberfläche verlaufen, beispielsweise unter einem Winkel von 25° bis 55° und besonders bevorzugt unter einem Winkel von 45°. Die Anordnung der Verbindung zwischen dem insertierbaren Element und dem Funktionsbauteil an einer Seite des Funktionsbauteils erleichtert die Insertion des insertierbaren Elements erheblich, im Gegensatz beispielsweise zu einer Insertion unterhalb des Funktionsbauteils. Gleichzeitig kann, durch die, vorzugsweise nach der Insertion erfolgende Verbindung des Schutzbauteils mit dem Funktionsbauteil dennoch gewährleistet werden, dass allseits um die Insertionsstelle herum, wie oben ausgeführt, ein mechanischer Schutz gewährleistet wird. Somit kann das oben beschriebene technische Dilemma gelöst werden, dass ein Raum für eine Insertion, beispielsweise eine Insertionshilfe, bereitgestellt werden kann, da während der Insertion lediglich das Funktionsbauteil im Bereich der Insertionsstelle und vorzugsweise neben der Insertionsstelle angeordnet ist. Nachträglich, also beispielsweise nach Entfernen der Insertionshilfe, kann dann das Schutzbauteil mit dem Funktionsbauteil verbunden werden und beispielsweise die Insertionsstelle vollständig umschließen, so dass allseitig ein Schutz der Insertionsstelle und vorzugsweise auch des in das Körpergewebe eingebrachten insertierbaren Elements gewährleistet werden kann. Die mindestens eine optionale Vertiefung des Funktionsbauteils kann die Funktionalität der Insertionshilfe erleichtern und beispielsweise in Verlängerung der Insertionsrichtung innerhalb des Körpergewebes ausgebildet sein.

Besonders bevorzugt ist es, wenn sowohl das Funktionsbauteil als auch das Schutzbauteil im verbundenen Zustand auf der Hautoberfläche direkt aufliegen. Zu diesem Zweck können sowohl das Funktionsbauteil als auch das Schutzbauteil jeweils mindestens eine Auflagefläche aufweisen. Dabei kann die Auflagefläche des Funktionsbauteils die Auflagefläche des Schutzbauteils zumindest teilweise, vorzugsweise vollständig umschließen. Die Auflageflächen können derart angeordnet sein, dass diese bei auf die Hautoberfläche aufgebrachter medizinischer Vorrichtung auf der Hautoberfläche aufliegen. Das Funktionsbauteil und das Schutzbauteil können jeweils mindestens eine der Hautoberfläche zuweisende selbstklebende Oberfläche zum Aufsetzen auf die Hautoberfläche und zum Verbinden mit der Hautoberfläche aufweisen. Beispielsweise können diese selbstklebenden Oberflächen in Form von Pflastern ausgestaltet sein oder in Form von einem oder mehreren Klebeschichten, welche auf die Oberflächen aufgebracht werden können. Das Funktionsbauteil und das Schutzbauteil können dabei jeweils separate und in auf die Hautoberfläche aufgesetztem Zustand abziehbare Liner aufweisen. Unter einem Liner ist dabei eine selbst vorzugsweise nicht klebende Schutzfolie zu verstehen, welche bei selbstklebenden Oberflächen aus dem Bereich der Pflastertechnik bekannt sind. Vorzugsweise können die Liner an einer Kante der selbstklebenden Oberfläche umgefaltet sein, so dass eine Abziehlasche entsteht. Diese Abziehlasche kann zunächst parallel zu einem auf der selbstklebenden Oberfläche aufgebrachten Teil des Liners verlaufen, beispielsweise unterhalb dieses Teils, zwischen dem auf der selbstklebenden Oberfläche aufgebrachten Teil und der Hautoberfläche. Die Abziehrichtung kann dann parallel zur selbstklebenden Oberfläche angeordnet sein, so dass der Abziehvorgang an der umgefalteten Kante beginnt und somit der Liner zuverlässig von der selbstklebenden Oberfläche abgezogen werden kann.

Besonders bevorzugt ist es, wenn das Funktionsbauteil und das Schutzbauteil jeweils eigene, nicht miteinander verbundene selbstklebende Oberflächen aufweisen. Alternativ können die selbstklebenden Oberflächen jedoch auch miteinander verbunden oder als mindestens eine gemeinsame selbstklebende Oberfläche ausgestaltet sein. Beispielsweise kann ein gemeinsames Pflaster für das Funktionsbauteil und das Schutzbauteil verwendet werden. Beispielsweise kann das Pflaster des Funktionsbauteils derart groß ausgestaltet werden, dass das Schutzbauteil mit auf dieses aufgebracht werden kann oder umgekehrt.

Wie oben ausgeführt, kann das Schutzbauteil als vollständig inertes Bauteil ausgestaltet sein und selbst außer einer mechanischen Stabilisierungs- und Schutzfunktion für das Funktionsbauteil und/oder den Sensor keinerlei eigene Funktionalität aufweisen. Das Schutzbauteil kann jedoch auch selbst mindestens eine weitere Funktionalität aufweisen. So kann das Schutzbauteil beispielsweise mindestens eine Durchführung aufweisen, insbesondere zur elektrischen Kontaktierung des Funktionsbauteils. Beispielsweise kann diese Durchführung mindestens eine einfache Öffnung zur Durchführung mindestens eines Kabels und/oder eines Steckers durch das Schutzbauteil hin zu dem Funktionsbauteil umfassen.

Wie oben dargestellt, umfasst das Gehäuse das mindestens eine Schutzbauteil und das mindestens eine Funktionsbauteil. Darüber hinaus kann das Gehäuse weitere Bauelemente umfassen. Beispielsweise kann das Gehäuse mindestens eine Schutzabdeckung aufweisen. Die Schutzabdeckung kann insbesondere zumindest teilweise transparent ausgestaltet sein, beispielsweise um eine visuelle Zugänglichkeit zu der Insertionsstelle zu gewährleisten. Die Schutzabdeckung kann als separates Bauteil ausgestaltet sein. Alternativ oder zusätzlich kann die Schutzabdeckung jedoch auch ganz oder teilweise Bestandteil des Funktionsbauteils und/oder des Schutzbauteils sein. Beispielsweise kann mindestens eine Schutzabdeckung vorgesehen sein, welche über das Schutzabbauteil und das Funktionsbauteil aufbringbar ist und im aufgebrachten Zustand das Schutzbauteil und das Funktionsbauteil zumindest teilweise auf einer der Hautoberfläche abgewandten Seite überdeckt. Beispielsweise kann die Schutzabdeckung als selbstklebendes Pflaster ausgestaltet sein, insbesondere als transparentes Pflaster. Auf diese Weise kann gewährleistet werden, dass durch das selbstklebende Pflaster hindurch beispielsweise eine Insertionsstelle von außen beobachtbar ist, insbesondere um beispielsweise Entzündungen detektieren zu können. Alternativ oder zusätzlich kann die mindestens eine Schutzabdeckung auch zumindest teilweise in das Schutzbauteil und/oder das Funktionsbauteil integriert sein. So ist beispielsweise eine Ausgestaltung der Schutzabdeckung als Kappe möglich, welche Bestandteil des Schutzbauteils sein kann. Beispielsweise kann das Schutzbauteil, wie oben ausgeführt, mindestens eine Aufnahme aufweisen, in welche das Funktionsbauteil eingefügt werden kann, also mindestens eine Öffnung. Diese Öffnung muss sich jedoch nicht notwendigerweise durchgehend von der Seite der Hautoberfläche zur gegenüberliegenden Seite des Schutzbauteils erstrecken. So kann die Öffnung auch lediglich von der Seite der Hautoberfläche her sich in einen Körper des Schutzbauteils hinein erstrecken, also beispielsweise in Form einer Vertiefung, insbesondere in Form einer auf das Funktionsbauteil angepassten Vertiefung. Auf der von der Hautoberfläche abgewandten Seite kann dann der verbleibende Rest des Schutzbauteils das Funktionsbauteil ganz oder teilweise überspannen und eine (beispielsweise fest mit dem Schutzbauteil verbundene) Schutzabdeckung des Funktionsbauteils bilden. Die Schutzabdeckung kann insbesondere in diesem Bereich transparent ausgestaltet sein.

Das Funktionsbauteil kann, wie oben dargestellt, mindestens eine medizinische Funktion aufweisen, also eingerichtet sein, um mindestens eine medizinische Funktion durchzuführen. Das Funktionsbauteil kann insbesondere mindestens eine mit dem insertierbaren Element verbindbare, insbesondere verbundene, elektronische und/oder elektromechanische und/oder fluidische Funktionskomponente umschließen. Beispielsweise kann es sich hierbei um einen elektronischen Baustein handeln, welcher ganz oder teilweise in dem Funktionsbauteil aufgenommen sein kann. Hierbei kann es sich beispielsweise um einen Verstärker oder einen Verstärkerbaustein handeln, beispielsweise einen Primärverstärker mit hoher Eingangsimpedanz.

Alternativ oder zusätzlich kann mindestens ein Potentiostat für elektrochemische Messungen vorgesehen sein. Wieder alternativ oder zusätzlich kann die mindestens eine Funktionskomponente beispielsweise mindestens eine Pumpe umfassen, beispielsweise eine Medikationspumpe.

Wie oben dargestellt, kann das Schutzbauteil insbesondere als inertes Schutzbauteil ausgestaltet sein, welches vorzugsweise lediglich mechanische Schutzfunktionen bereitstellt. Es ist jedoch ebenso möglich, dass auch das Schutzbauteil selbst mindestens eine Funktion aufweist, beispielsweise eine elektrische Funktion, eine elektronische Funktion, eine Datenspeicherfunktion, eine Energieversorgungsfunktion, eine Kommunikationsfunktion, eine Messfunktion, eine Medikationsfunktion, eine Aktorfunktion, eine Sensorfunktion oder eine Kombination der genannten und/oder anderer Funktionen. Beispielsweise kann das Schutzbauteil mindestens eine elektronische und/oder elektromechanische und/oder fluidische Funktionskomponente umschließen, beispielsweise analog zur oben beschriebenen Ausgestaltung des Funktionsbauteils. Die Funktionskomponenten des Funktionsbauteils und des Schutzbauteils können dabei unabhängig voneinander ausgestaltet sein, können jedoch auch zusammenwirken. Beispielsweise kann das Funktionsbauteil mindestens eine elektronische Komponente umfassen, welche mit einem insertierbaren Element in Form eines Sensors zusammenwirkt. Das Schutzbauteil kann mindestens eine fluidische Funktionskomponente umfassen, beispielsweise in Form einer Medikationspumpe, welche mit einem weiteren insertierbaren Element zusammenwirken kann, beispielsweise einer Kanüle, um eine Medikationsfunktion auszuüben. So kann, zusätzlich zu dem mindestens einen insertierbaren Element, welches mit dem Funktionsbauteil verbindbar oder verbunden ist, mindestens ein weiteres insertierbares Element vorgesehen sein, welches mit dem Schutzbauteil verbindbar und/oder verbunden ist. Beispielsweise kann es sich hierbei wiederum um eines oder mehrere der oben beschriebenen insertierbaren Elemente handeln, so dass beispielsweise eine Sensorfunktion auch mit einer Medikationsfunktion oder einer anderen Aktorfunktion kombiniert werden kann, wobei die Funktionen beispielsweise auf das Schutzbauteil und das Funktionsbauteil verteilbar sind. Das Schutzbauteil kann insbesondere eine Funktionskomponente umschließen, welche ausgewählt sein kann aus: einem Energiespeicher, insbesondere einer Batterie; einem Datenspeicher, insbesondere einem nicht-flüchtigen Datenspeicher; einem Kommunikationsbaustein für drahtlose und/oder drahtgebundene Kommunikation mit mindestens einem externen Gerät, insbesondere einem Funkbaustein; einer Medikationspumpe. Auch Kombinationen der genannten und/oder anderer Funktionskomponenten sind denkbar.

Darüber hinaus kann die Vorrichtung ein oder mehrere weitere Funktionen und/oder Funktionskomponenten umfassen. Beispielsweise kann in dem Schutzbauteil und/oder in dem Funktionsbauteil mindestens eine saugfähige Komponente vorgesehen sein, beispielsweise um an der Insertionsstelle austretende Körperflüssigkeit aufzunehmen. Besonders bevorzugt ist es, wenn eine derartige saugfähige Komponente in dem Schutzbauteil vorgesehen ist, da dieses in der Regel einfach ausgetauscht und entsorgt werden kann. Die saugfähige Komponente kann beispielsweise mindestens ein saugfähiges textiles und/oder mindestens ein saugfähiges Papiermaterial umfassen. Alternativ oder zusätzlich sind auch andere saugfähige Materialien einsetzbar, beispielsweise saugfähige schwammartige Materialien, beispielsweise aus einem hydrophilen Kunststoff oder Naturstoff.

Wie oben ausgeführt, sind das Funktionsbauteil und das Schutzbauteil miteinander verbindbar, vorzugsweise ausschließlich mechanisch. Darüber hinaus kann das Gehäuse jedoch auch mindestens eine elektrische Schnittstelle zwischen dem Funktionsbauteil und dem Schutzbauteil aufweisen, wobei die Schnittstelle derart eingerichtet sein kann, dass bei der Verbindung zwischen dem Funktionsbauteil und dem Schutzbauteil auch eine elektrische Verbindung zwischen dem Funktionsbauteil und dem Schutzbauteil herstellbar ist. Beispielsweise kann eine elektrische Steckverbindung hergestellt werden, welche vorzugsweise reversibel ausgestaltet ist. Auf diese Weise können beispielsweise Funktionskomponenten innerhalb des Schutzbauteils mit Funktionskomponenten innerhalb des Funktionsbauteils miteinander kommunizieren und/oder Daten und/oder Energie austauschen. Besonders bevorzugt ist es jedoch, wie oben ausgestaltet, wenn mindestens eine Funktion des Funktionsbauteils unabhängig von dem Vorhandensein des Schutzbauteils ausübbar ist, so dass beispielsweise ohne Vorhandensein des Schutzbauteils eine Energieversorgung innerhalb des Funktionsbauteils besteht, beispielsweise durch einen Energiespeicher. Alternativ kann eine elektrische Energie jedoch auch durch das Schutzbauteil bereitgestellt werden.

In einem weiteren Aspekt der vorliegenden Erfindung wird mindestens ein Schutzbauteil zum Einsatz in einer medizinischen Vorrichtung mit mindestens einem Funktionsbauteil und mindestens einem in ein Körpergewebe eines Benutzers insertierbaren, mit dem Funktionsbauteil verbindbaren Element vorgeschlagen. Insbesondere kann es sich dabei um eine medizinische Vorrichtung gemäß einer oder mehreren der oben beschriebenen Ausgestaltungen handeln, so dass für bevorzugte Ausführungen des Schutzbauteils auf die obige Beschreibung und die dort dargestellten optionalen, das Schutzbauteil betreffenden Merkmale verwiesen werden kann. Auch eine andere Ausgestaltung ist jedoch grundsätzlich möglich. Das Schutzbauteil weist mindestens eine Auflagefläche zur Auflage auf einer Hautoberfläche des Benutzers auf. Das Schutzbauteil ist mit dem Funktionsbauteil verbindbar, wobei das Schutzbauteil mindestens eine Aufnahme mit mindestens einer Öffnung aufweist, wobei die Aufnahme derart eingerichtet ist, dass das Funktionsbauteil in die Aufnahme einbringbar ist. Das Schutzbauteil umschließt dabei das Funktionsbauteil in verbundenem Zustand zumindest teilweise. Beispielsweise können ein Funktionsbauteil und ein oder mehrere Schutzbauteile auch zu einem Kit zusammengefasst und als Kit auslieferbar sein. Das Kitt kann weiterhin ein oder mehrere insertierbare Elemente identischer oder unterschiedlicher Art umfassen. Insbesondere kann das Schutzbauteil in der medizinischen Vorrichtung, wie oben beschrieben, austauschbar ausgestaltet sein, so dass beispielsweise während des Betriebs der medizinischen Vorrichtung das Schutzbauteil ausgetauscht werden kann gegen ein neues Schutzbauteil, beispielsweise zu hygienischen Zwecken.

In einem weiteren Aspekt der vorliegenden Erfindung wird ein Verfahren zum Schutz mindestens eines auf eine Hautoberfläche eines Benutzers aufgebrachten Funktionsbauteils einer medizinischen Vorrichtung und mindestens eines mit dem Funktionsbauteil verbundenen, in ein Körpergewebe des Benutzers insertierbaren, insbesondere insertierten, Elements vorgeschlagen. Auch hierbei kann wiederum eine medizinische Vorrichtung gemäß einer oder mehreren der obigen Ausgestaltungen verwendet werden, so dass bezüglich optionaler Ausgestaltungen der medizinischen Vorrichtung auf die obige Beschreibung verwiesen werden kann. Insbesondere kann ein erfindungsgemäßes Schutzbauteil eingesetzt werden. Bei dem vorgeschlagenen Verfahren wird mindestens ein Schutzbauteil mit dem Funktionsbauteil verbunden, wobei das Schutzbauteil das Funktionsbauteil zumindest teilweise umschließt.

Das Verfahren kann insbesondere auf eine Anordnung angewandt werden, bei welcher das insertierbare Element bereits in das Körpergewebe insertiert und mit dem Funktionsbauteil verbunden ist. Die Insertion kann dabei Bestandteil des Verfahrens sein, ist jedoch vorzugsweise nicht Bestandteil dieses Verfahrens. Bei einer derartigen Anordnung kann optional ein Liner von einer selbstklebenden Oberfläche des Funktionsbauteils entfernt werden, so dass das Funktionsbauteil auf der Hautoberfläche haftet. Anschließend, gleichzeitig oder zuvor kann das Schutzbauteil mit dem Funktionsbauteil verbunden werden, beispielsweise indem das Funktionsbauteil in eine Aufnahme mit mindestens einer Öffnung des Schutzbauteils eingebracht wird, vorzugsweise einer durchgehenden Öffnung. Anschließend kann optional ein Liner von einer selbstklebenden Oberfläche des Schutzbauteils entfernt werden, so dass das Schutzbauteil auf der Hautoberfläche fixiert ist und vorzugsweise auch räumlich zu dem Funktionsbauteil fixiert ist, durch die Verbindung mit dem Funktionsbauteil und/oder das Aufkleben auf die Hautoberfläche. Optional kann in einem weiteren Verfahrensschritt eine Schutzabdeckung über das Schutzbauteil und das Funktionsbauteil aufgebracht werden, beispielsweise eine flexible und/oder verformbare Schutzabdeckung, beispielsweise in Form einer transparenten Folie, insbesondere einer selbstklebenden Folie, vorzugsweise in Form eines transparenten Pflasters. Diese Schutzabdeckung kann selbst in einem Bereich um das Funktionsbauteil und/oder das Schutzbauteil herum auf die Hautoberfläche aufgeklebt werden oder kann lediglich auf das Funktionsbauteil und das Schutzbauteil aufgeklebt werden. Verschiedene Ausgestaltungen sind möglich.

Die vorgeschlagenen Vorrichtungen und das vorgeschlagene Verfahren weisen gegenüber bekannten Vorrichtungen und Verfahren zahlreiche Vorteile auf. Insbesondere kann erfindungsgemäß eine Sensorbasis, welche mit dem insertierbaren Element zusammenwirkt, in mehrere Einzelteile aufgeteilt werden, welche nacheinander montiert werden können und optional auch aus verschiedenen Werkstoffen bestehen können. Diese Einzelteile, welche das Funktionsbauteil und das Schutzbauteil umfassen, können in ihrer Größe und Form dem jeweiligen Arbeitsschritt angepasst sein. Insbesondere nach Abschluss der Insertionsprozedur können die montierten Einzelteile, also das Funktionsbauteil und das Schutzbauteil sowie optional weitere Bauteile, eine Einheit bilden. Zum Zeitpunkt der Insertion kann die Sensorbasis lediglich aus einem möglichst klein konstruierten Teil bestehen, nämlich dem Funktionsbauteil, welches nur die notwendigsten mechanischen Anforderungen erfüllen kann und aus einem für diesen Zweck optionalen Werkstoff hergestellt sein kann. Der Werkstoff des Funktionsbauteils kann beispielsweise feuchtigkeitsdicht ausgestaltet sein und/oder kann elektrisch abschirmende Eigenschaften aufweisen und/oder kann eine hohe mechanische Festigkeit aufweisen. Aufgrund des kleinen Bauraums des Funktionsbauteils kann eine optimale Insertionsvorrichtung zur Insertion des insertierbaren Elements realisiert werden. Weiterhin ist ein optimaler Zugang zur Wunde, also zur Insertionsstelle, möglich, wodurch die Beobachtung und Reinigung derselben ideal durchgeführt werden kann. Schutzfunktionen für elektronische Komponenten können durch geeignete Gehäusewerkstoffe des Funktionsbauteils, die zumeist keine Elastizität besitzen müssen, realisiert werden. Beispielsweise nach der Insertion kann dann ein mechanischer Schutz montiert werden, in Form des Schutzbauteils, welcher sich durch eine oder mehrere mechanische und optional auch durch eine oder mehrere Schnittstellen mit dem Funktionsbauteil verbinden kann. Dieser Schutz kann nun in einer idealen Größe und aus speziellen Werkstoffen ausgeführt werden, da kein Kompromiss mit der Insertionsvorrichtung oder der Elektronikumhausung des Funktionsbauteils eingegangen werden muss. So ist beispielsweise ein ringförmig um eine Insertionsstelle und/oder Insertionsvorrichtung umlaufender mechanischer Schutz durch das Schutzbauteil realisierbar. Als Werkstoffe für das Schutzbauteil sind insbesondere elastische Silikone oder andere hautverträgliche Werkstoffe möglich. Die Verbindung des Schutzbauteils mit dem Funktionsbauteil kann beispielsweise mittels eines Kraft- und/oder Formschlusses erfolgen. Bei einem Nachbluten der Wunde im Bereich der Insertionsstelle während der Tragedauer ist es in der Regel ausreichend, den äußeren, mechanischen Schutz durch das Schutzbauteil zu entfernen und die Wunde zu reinigen und/oder zu kontrollieren. Die eigentliche medizinische Funktion, beispielsweise der eigentliche Sensor bzw. das insertierbare Element, kann platziert bleiben, und die Platzverhältnisse zur Reinigung und Kontrolle sind dennoch ideal ausführbar. Nach erfolgter Reinigung und/oder Kontrolle kann der Schutz wieder montiert werden. Die Reinigung kann vor der Verbindung des Schutzbauteils mit dem Funktionsbauteil und/oder auch erst nach Herstellung dieser Verbindung erfolgen.

Weiterhin ist es auch möglich, neben der mindestens einen mechanischen Funktion andere Systemnotwendigkeiten in die aufgeteilten Bauteile des Gehäuses zu verteilen. So kann beispielsweise eine Unterbringung einer Antenne, eines Energiespeichers oder eine Kabelzugentlastung in verschiedenen Einzelteilen möglich sein, beispielsweise in dem Funktionsbauteil und/oder in dem Schutzbauteil.

Die Möglichkeit, eine schrittweise Montage und Verbindung der Einzelteile zu realisieren, erlaubt auch die räumliche Integration unabhängiger Zweitsysteme in eine Basis der medizinischen Vorrichtung, beispielsweise in eine Sensorbasis, insbesondere eine Sensorbasis eines kontinuierlich messenden Sensors. So ist es beispielsweise möglich, eine Medikationsfunktion, beispielsweise einen Infusionskatheter zur Insulindosierung und/oder eine Insulinpumpe oder Teile der genannten Vorrichtungen in aufgeteilte Bauteile zu integrieren. Die Einzelteile können getrennt entwickelt und gefertigt werden und werden beispielsweise beim Kunden zusammengefügt. So kann die medizinische Vorrichtung beispielsweise ein erstes insertierbares Element in Form eines Sensors, beispielsweise eines kontinuierlichen messenden Sensors, gekoppelt oder koppelbar mit dem Funktionsbauteil, in Kombination mit einem zweiten insertierbaren Element in Form eines Infusionskatheters, gekoppelt mit dem Schutzbauteil, realisieren, wobei beispielsweise das Schutzbauteil mindestens eine Medikationsfunktion aufweist, beispielsweise einen fluidischen und/oder elektromechanischen Baustein zur Medikation, beispielsweise eine Insulinpumpe oder eine andere Art von Medikationspumpe.

Die medizinische Vorrichtung kann beispielsweise derart angewandt werden, dass zunächst das mindestens eine insertierbare Element in das Körpergewebe insertiert wird. Beispielsweise kann es sich hierbei um einen Sensor handeln. Alternativ oder zusätzlich können ein oder mehrere weitere insertierbare Elemente insertiert werden, beispielsweise eine Kanüle einer Medikationspumpe, beispielsweise einer Insulinpumpe. Erfindungsgemäß kann eine Schutzvorrichtung für den bereits insertierten Sensor bzw. das bereits insertierte Element bereitgestellt werden. So kann beispielsweise zunächst das Funktionsbauteil mit dem insertierbaren Element gekoppelt werden oder bereits während der Insertion mit dem Funktionsbauteil gekoppelt sein. Das Schutzbauteil wird dann vorzugsweise, nachdem das insertierbare Element mit Hilfe beispielsweise einer Insertionshilfe in das Körpergewebe eingebracht wurde und vorzugsweise nachdem das Funktionsbauteil mit der Hautoberfläche verbunden wurde, beispielsweise über die selbstklebende Oberfläche und/oder ein Pflaster, mit dem Funktionsbauteil verbunden. Dies kann beispielsweise dadurch erfolgen, dass das Schutzbauteil über das Funktionsbauteil derart gestülpt wird, dass immer noch die Insertionsstelle, beispielsweise die Einstichstelle, erkennbar ist und beispielsweise für den Benutzer oder einen Arzt oder Pflegepersonal zugänglich ist, um beispielsweise überschüssiges Blut aufzunehmen oder um eine langfristige Beobachtung der Insertionsstelle vorzunehmen. Der Sinn des Schutzbauteils kann insbesondere darin bestehen, einen mechanischen Schutz bereitzustellen, um zum einen die Lage des insertierbaren Elements während des Tragens, beispielsweise des Sensors während eines 7-tägigen Tragens, stabil zu halten und um Stöße und/oder Verschiebungen der Haut von dem insertierbaren Element abzuhalten. Hierbei hat es sich als besonders günstig herausgestellt, wenn die Insertionsstelle in der Mitte des Gehäuses angeordnet ist, also mittig bezüglich der Fläche, auf welcher das Gehäuse auf der Hautoberfläche aufliegt. Das Schutzbauteil kann beispielsweise in Form eines Rings, beispielsweise eines runden und/oder ovalen Schwimmrings, ausgestaltet sein, der, beispielsweise in Form eines wasserdichten Pflasters, oberhalb des insertierbaren Elements, beispielsweise des Sensors agiert. Darüber hinaus kann es vorteilhaft sein, wenn die vollständige Lage des insertierbaren Elements und/oder des Funktionsbauteils durch das Schutzbauteil abgedeckt sind. Bevorzugte Ausgestaltungen für das Schutzbauteil sind Ausgestaltungen mit flexiblen Werkstoffen, im Gegensatz zu den bevorzugten harten Werkstoffen, welche für das Funktionsbauteil eingesetzt werden können. Das Schutzbauteil kann vorzugsweise jederzeit entfernbar sein, also insbesondere von der Funktion des Funktionsbauteils, in Zusammenwirkung mit dem insertierbaren Element, entkoppelbar sein, beispielsweise von einer Sensorfunktion des Funktionsbauteils und des insertierbaren Sensors. Bei langfristiger Anwendung des insertierbaren Elements kann beispielsweise das Schutzbauteil aus hygienischen Gründen austauschbar ausgestaltet sein.

### Kurze Beschreibung der Figuren

Weitere Einzelheiten und Merkmale der Erfindung ergeben sich aus der nachfolgenden Beschreibung von bevorzugten Ausführungsbeispielen, insbesondere in Verbindung mit den Unteransprüchen. Hierbei können die jeweiligen Merkmale für sich alleine oder zu mehreren in Kombination miteinander verwirklicht sein. Die Erfindung ist nicht auf die Ausführungsbeispiele beschränkt. Die Ausführungsbeispiele sind in den Figuren schematisch dargestellt. Gleiche Bezugsziffern in den einzelnen Figuren bezeichnen dabei gleiche oder funktionsgleiche beziehungsweise hinsichtlich ihrer Funktionen einander entsprechende Elemente.

Im Einzelnen zeigen:
- Figuren 1A und 1B: verschiedene Ansichten eines Funktionsbauteils und eines mit dem Funktionsbauteil verbundenen insertierbaren Elements vor einer Insertion in das Körpergewebe;
- Figuren 2A und 2B: verschiedene Ansichten der Baugruppe gemäß den Figuren 1A und 1B nach Abziehen eines Liners und nach Insertion in ein Körpergewebe;
- Figuren 3A und 3B: verschiedene Ansichten eines Schutzbauteils;
- Figur 4: eine Ansicht einer medizinischen Vorrichtung nach Aufsetzen des Schutzbauteils gemäß den Figuren 3A und 3B auf das Funktionsbauteil gemäß den Figuren 2A und 2B und vor Abziehen eines Liners; und
- Figuren 5A und 5B: verschiedene Ansichten der medizinischen Vorrichtung gemäß Figur 4 nach Abziehen des Liners von dem Schutzbauteil.

### Ausführungsbeispiele

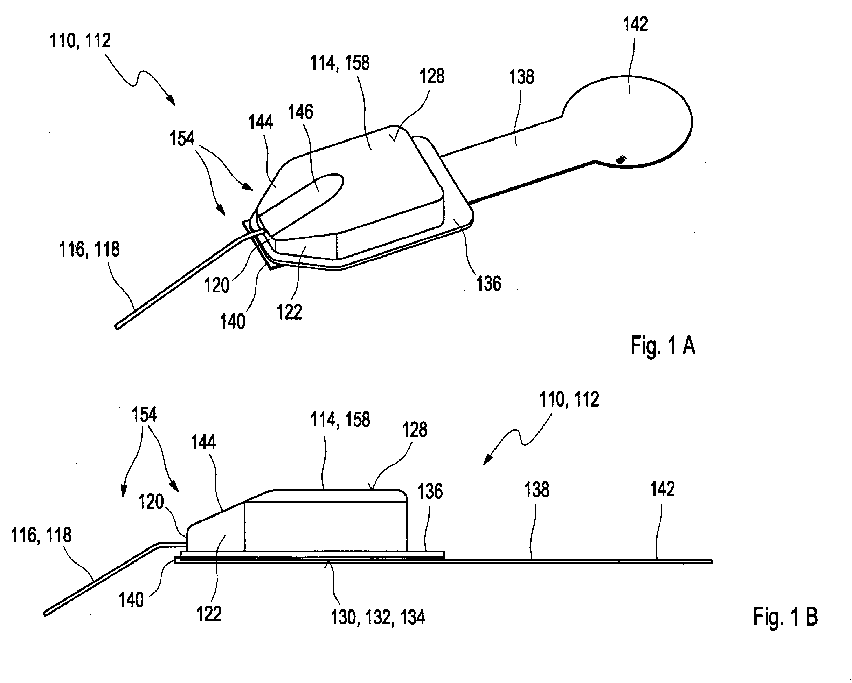

In den Figuren 1A bis 5B sind verschiedene Komponenten einer medizinischen Vorrichtung 110 zur Ausübung mindestens einer medizinischen Funktion in verschiedenen Ansichten und in verschiedenen Stufen einer Zusammensetzung dargestellt. In dem dargestellten Ausführungbeispiel handelt es sich bei der medizinischen Vorrichtung um eine Sensorvorrichtung 112, welche zur Langzeitmessung eines oder mehrerer medizinischer Parameter, insbesondere zur Langzeitmessung eines oder mehrerer Analyten in einem Körpergewebe und/oder einer Körperflüssigkeit, beispielsweise zur Langzeitüberwachung einer Glukosekonzentration, eingesetzt werden kann. Gleichzeitig zeigen die Figuren 1A - 5B verschiedene mögliche Stufen eines Ausführungsbeispiels eines erfindungsgemäßen Verfahrens zum Schutz mindestens eines auf einer Hautoberfläche eines Benutzers aufgebrachten Funktionsbauteils 114 und eines mit dem Funktionsbauteil 114 verbundenen insertierbaren Elements 116, welches in dem dargestellten Ausführungsbeispiel als insertierbarer Sensor 118 ausgestaltet ist. Alternativ oder zusätzlich können jedoch auch andere Arten von insertierbaren Elementen vorgesehen sein.

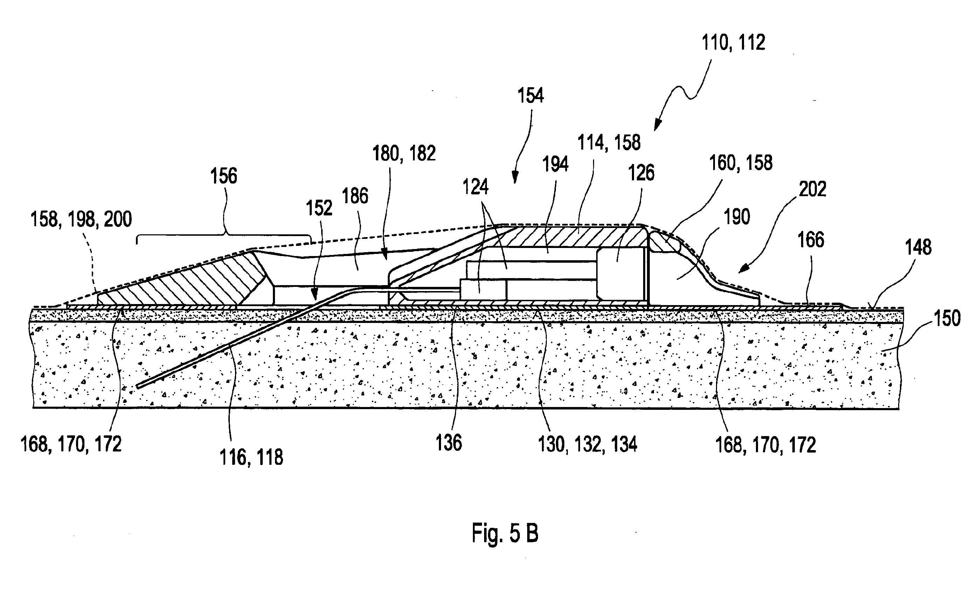

Das Funktionsbauteil 114 kann, wie in Figur 1A dargestellt, insbesondere einteilig ausgestaltet sein. Wie oben beschrieben, kann das Funktionsbauteil 114 jedoch grundsätzlich auch mehrteilig ausgestaltet sein und kann beispielsweise mehrere Funktionsbauteil-Komponenten umfassen, beispielsweise ein Disposable und ein Reusable. Das Funktionsbauteil 114 ist in dem dargestellten Ausführungsbeispiel im Wesentlichen quaderförmig ausgestaltet, mit einer Stirnseite 120 und zu dieser Stirnseite 120 hin weisenden abgeschrägten Kanten 122. Durch die abgeschrägten Kanten 122 stellt in dem dargestellten Ausführungsbeispiel die Stirnseite 120 die schmalste Seite des Grundrisses des Funktionsbauteils 114 dar, bei einer Draufsicht von oben auf das Funktionsbauteil 114 in Figur 1B. Das insertierbare Element 116 ist in den Darstellungen gemäß den Figuren 1A und 1B an der Stirnseite 120 mit dem Funktionsbauteil 114 verbunden. Beispielsweise kann das insertierte Element 116 direkt mit dem Funktionsbauteil 114 verbunden sein oder ins Innere des Funktionsbauteils 114 geführt sein und dort beispielsweise mit einer in den Figuren 1A und 1B nicht erkennbaren, in einer Schnittdarstellung gemäß Figur 5B durch die medizinische Vorrichtung 110 jedoch erkennbaren Funktionskomponente 124 verbunden sein, beispielsweise eine Ansteuer- und Auswerteschaltung, beispielsweise mit mindestens einem Potentiostaten und/oder mindestens einem hochohmigen Messverstärker. Diese mindestens eine Funktionskomponente 124 kann beispielsweise über mindestens eine Schnittstelle 126, wie ebenfalls in Figur 5B erkennbar, kontaktierbar sein. Diese Schnittstelle 126 kann beispielsweise eine Kabeldurchführung und/oder einen Stecker und/oder eine ähnliche Art zur elektrischen Kontaktierung der Funktionskomponente 124 und/oder zur Energieversorgung und/oder zur Ansteuerung der Funktionskomponente 124 umfassen.

Das Funktionsbauteil 114 weist, wie in Figur 1B erkennbar, eine Oberseite 128 und eine Unterseite 130 auf. Im Betrieb weist die Oberseite 128 von einer Hautoberfläche eines Benutzers weg, wohingegen die Unterseite 130 der Hautoberfläche zuweist. Die Unterseite 130 ist dementsprechend als Auflagefläche 132 des Funktionsbauteils 114 ausgestaltet. Diese Auflagefläche 132 ist in dem dargestellten Ausführungsbeispiel als selbstklebende Oberfläche 134 ausgestaltet, beispielsweise mittels eines selbstklebenden Pflasters 136, wie in der perspektivischen Draufsicht gemäß Figur 1A erkennbar ist. Die selbstklebende Oberfläche 134 ist in dem dargestellten Ausführungsbeispiel durch einen abziehbaren Liner 138 geschützt, also einer abziehbaren Schutzfolie, welche die selbstklebende Oberfläche 134 im unbenutzten Zustand vollständig bedeckt. Exemplarisch ist dieser Liner 138 an einer Kante 140 umgebogen und parallel zu sich selbst zurückgeführt, so dass eine Abziehlasche 142 auf der der Stirnseite 120 gegenüberliegenden Seite entsteht.

Die Baugruppe gemäß den Figuren 1A und 1B umfasst das Funktionsbauteil 114 und das insertierbare Element 116, wird in dem dargestellten Zustand auf eine Hautoberfläche eines Benutzers aufgesetzt, und das insertierbare Element 116 wird mittels einer geeigneten, nicht dargestellten Insertionsvorrichtung (Serter) in das Körpergewebe des Patienten insertiert. Um das Aufsetzen der Insertionsvorrichtung, beispielsweise mit einer Kanüle, in der das insertierbare Element 116 ganz oder teilweise aufgenommen ist, während der Insertion, räumlich zu erleichtern, verläuft die Oberseite 128 des Funktionsbauteils 114 zur Stirnseite 120 abgeschrägt. Auch der insertierbare Sensor 118 in den in den Figuren 1A und 1B dargestellten Ausführungsbeispielen verläuft schräg zur Unterseite 130, so dass dieser bei der Insertion schräg in das Körpergewebe eindringt, beispielsweise unter einem Winkel von ca. 45°. Der insertierbare Sensor 118 kann auch beispielsweise gekrümmt verlaufen. Beispielsweise kann der insertierbare Sensor zunächst außerhalb des Körpergewebes im Wesentlichen gerade verlaufen oder mit einer ersten Krümmung verlaufen und dann unter einem Winkel, beispielsweise dem genannten Winkel von 45°, in das Körpergewebe eindringen. Innerhalb des Körpergewebes kann dann der insertierbare Sensor 118 beispielsweise in einer anderen Richtung und/oder mit einer anderen Krümmung oder auch beispielsweise gerade und/oder unter einem anderen Winkel zur Hautoberfläche verlaufen als außerhalb des Körpergewebes. So kann beispielsweise der insertierbare Sensor 118 innerhalb des Körpergewebes unter einem flacheren Winkel zur Hautoberfläche verlaufen als außerhalb des Körpergewebes. So kann der insertierbare Sensor 118 beispielsweise innerhalb des Körpergewebes unter einem Winkel von weniger als 20° zur Hautoberfläche verlaufen oder sogar im Wesentlichen parallel zur Hautoberfläche. Die Abschrägung 144 der Oberseite 128 hin zur Stirnseite 120 kann im Wesentlichen dem Winkel des insertierbaren Sensors 118 zur Hautoberfläche außerhalb des Körpergewebes folgen. Weiterhin kann das Funktionsbauteil 114 eine längliche Vertiefung 146 aufweisen, beispielsweise eine Nut, in welcher die Insertionsnadel oder ein anderer Teil der Insertionsvorrichtung verlaufen kann und welche ebenfalls von ihrem Winkel her dem Winkel des insertierbaren Elements 116 im Wesentlichen folgen kann. Auf diese Weise kann das Funktionsbauteil 114 derart ausgestaltet sein, dass dieses räumlich die Insertion nicht oder geringstmöglich behindert.

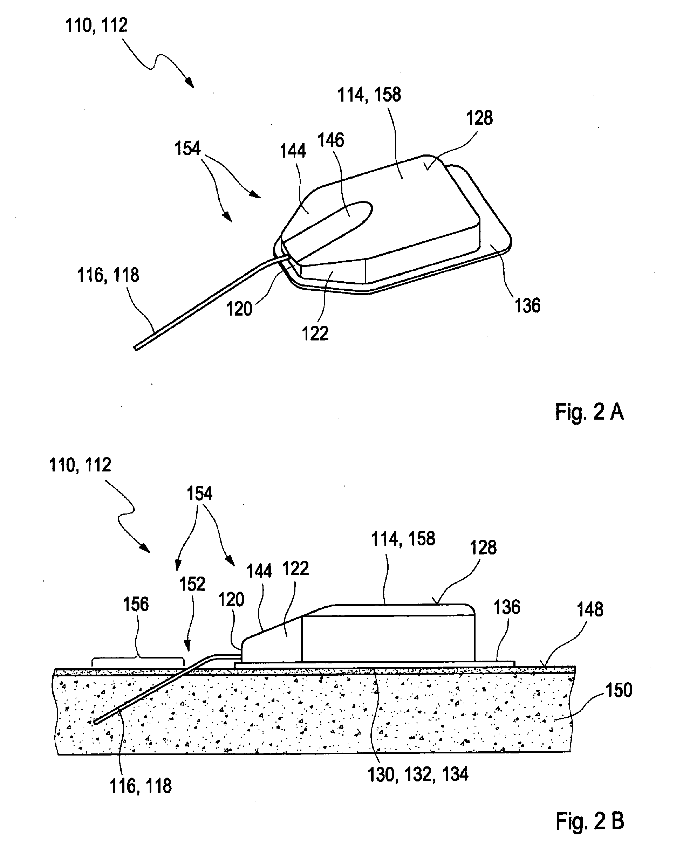

Nach dem Aufsetzen auf die Hautoberfläche und der Insertion kann der Liner 138 entfernt werden, beispielsweise indem die Abziehlasche 142 in eine Richtung von der Kante 140 weggezogen wird. Die selbstklebende Oberfläche 134 klebt nun auf der Hautoberfläche. Dies ist in den Figuren 2A und 2B dargestellt, wobei Figur 2A eine Draufsicht auf die Sensorbaugruppe mit dem Funktionsbauteil 114 und dem insertierbaren Element 116 nach Abziehen des Liners 138 zeigt und wobei Figur 2B eine Seitenansicht der Sensorbaugruppe zeigt, gemeinsam mit der Hautoberfläche 148 und einem Schnitt durch das Körpergewebe 150. Gemäß Figur 2B ist erkennbar, dass das insertierbare Element 118 an einer Insertionsstelle 152 die Hautoberfläche 148 durchdringt. Beispielsweise kann diese Insertionsstelle 152 im Wesentlichen am Ort eines virtuellen Schnittpunkts des insertierbaren Elements 116 im Ruhezustand, also im nichtinsertierten Zustand, mit einer Ebene der Unterseite 130 angeordnet sein, sofern das insertierbare Element 116 bei der Insertion nicht oder nur unwesentlich verformt wird. Beispielsweise kann eine Abweichung um nicht mehr als 3 mm gegeben sein, vorzugsweise um nicht mehr als 1 mm, um Spannungen zu vermeiden.

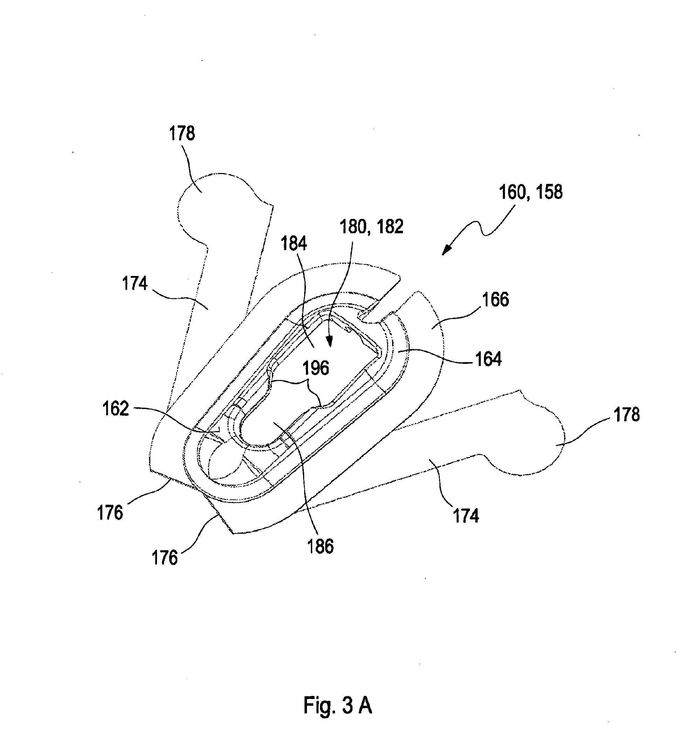

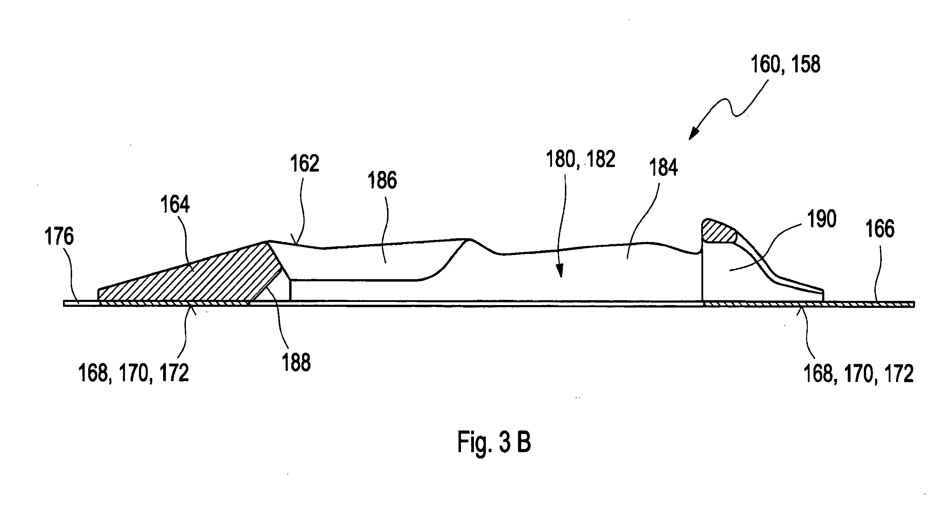

Das insertierbare Element 116 und das Funktionsbauteil 114 bilden eine Funktionsbaugruppe 154. Wie oben beschrieben, ist das Funktionsbauteil 114 derart klein dimensioniert und derart relativ zur Insertionsstelle 152 ausgestaltet, dass eine optimale Insertion möglich ist. Hierdurch ergeben sich jedoch in dem in Figur 2B dargestellten insertierten Zustand des insertierbaren Elements 116 verschiedene Probleme. So kann sich das Funktionsbauteil 114 auf der Hautoberfläche 148 relativ zur Insertionsstelle 152 verschieben, was zu schmerzhaften Spannungen oder sogar Verletzungen im Bereich der Insertionsstelle 152 führen kann. Weiterhin ist ein Insertionsbereich 156 auf der Hautoberfläche 148, welcher eine Projektion des insertierbaren Elements 116 im insertierten Zustand auf die Hautoberfläche 148 ist, sowie umgebende, benachbarte Bereiche, sehr empfindlich gegenüber Druck, Zug oder ähnlichen mechanischen Belastungen. Derartige mechanische Belastungen können die Messergebnisse des insertierbaren Sensors 118 beeinflussen und/oder können äußerst schmerzhaft sein. Mit diesen Nachteilen wurde die freie Zugänglichkeit der Insertionsstelle 152 durch die entsprechende einseitige und kleine Ausgestaltung des Funktionsbauteils 114 erkauft. Um dennoch den Insertionsbereich 156 und die Insertionsstelle 152 optimal zu schützen, wird vorgeschlagen, dass das Funktionsbauteil 114 lediglich ein Teil eines mehrteiligen Gehäuses 158 ist. Es wird dementsprechend ein Verfahren zum Schutz der Funktionsbaugruppe 154 vorgeschlagen, bei welchem die Funktionsbaugruppe 154 durch ein zusätzliches Schutzbauteil 160 geschützt ist. Dieses Schutzbauteil 160 als Teil des mehrteiligen Gehäuses 158 ist in den Figuren 3A und 3B in verschiedenen Ansichten gezeigt. Dabei zeigt Figur 3A eine perspektivische Draufsicht auf eine im Betrieb von der Hautoberfläche 148 weg weisende Oberseite 162, und Figur 3B zeigt eine Schnittdarstellung durch das Schutzbauteil 160 von der Seite. Wie aus der Schnittdarstellung gemäß Figur 3B erkennbar ist, umfasst das Schutzbauteil 160 ein Schutzgehäuse 164, auf dessen Unterseite wiederum ein Pflaster 166 angeordnet ist. Dementsprechend entsteht auf mindestens einer Unterseite 168 des Schutzbauteils 160 mindestens eine Auflagefläche 170, welche als selbstklebende Oberfläche des Schutzbauteils 160 ausgestaltet ist. Beispielsweise können diese Auflagefläche 170 und diese selbstklebende Oberfläche 172 im zusammengesetzten Zustand des Gehäuses 158 die Auflagefläche 132 des Funktionsbauteils 114 teilweise oder vorzugsweise sogar vollständig umschließen. Die selbstklebende Oberfläche 172 kann, wie in Figur 3 dargestellt, wiederum durch einen oder mehrere Liner 174 geschützt sein, welche in Figur 3B nicht dargestellt sind. Beispielsweise kann das Pflaster 166 wiederum eine oder mehrere Kanten 176 aufweisen, an welchen der Liner 174 umgebogen und parallel zu sich selbst zurückgeführt ist, so dass wiederum eine oder mehrere Abziehlaschen 178, vorzugsweise zwei Abziehlaschen 178, entstehen. Durch Ziehen der Abziehlaschen 178 von den Kanten 176 weg wird die selbstklebende Oberfläche 172 des Schutzbauteils 160 freigelegt und beispielsweise mit einer in den Figuren 3A und 3B nicht dargestellten Hautoberfläche 148 verklebt.

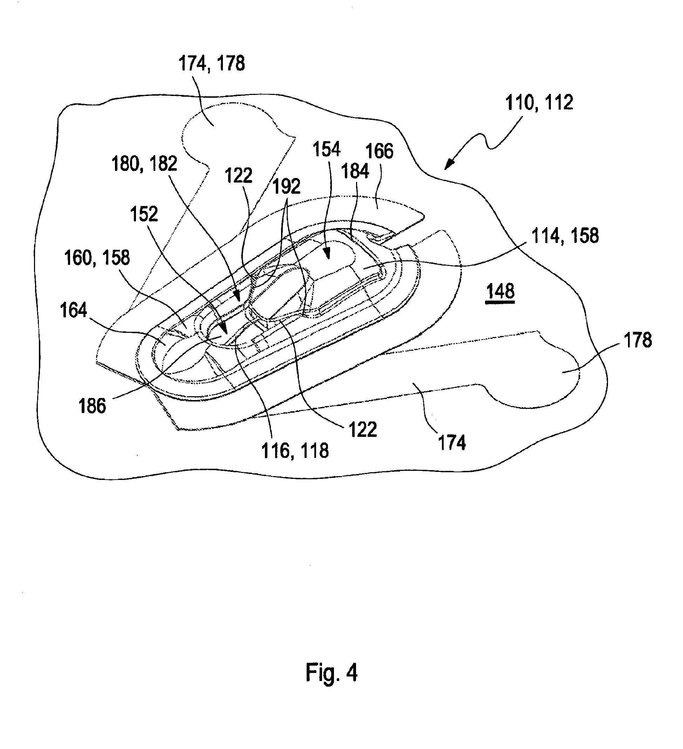

Weiterhin weist das Schutzbauteil 160 eine Aufnahme 180 in Form einer Öffnung 182 auf. Bei dem vorgeschlagenen Verfahren zum Schutz der Funktionsbaugruppe 154, vorzugsweise bei bereits in das Körpergewebe 150 insertiertem Element 116, wird das Schutzbauteil 160 über das Funktionsbauteil 114 gestülpt, indem das Funktionsbauteil 114 in diese Öffnung 182 eingefügt wird. Es kann beispielsweise ein Kraft- und/oder Formschluss zwischen dem Schutzbauteil 160 und dem Funktionsbauteil 114 entstehen. Dies ist in Figur 4 dargestellt. Das Schutzbauteil 160 umschließt dabei in Draufsicht auf die Hautoberfläche das Funktionsbauteil 114 und vorzugsweise auch den Insertionsbereich 156 (in Figur 4 nicht dargestellt) teilweise oder vollständig. Vorzugsweise weist die Öffnung 182 gemäß Figuren 3A und 3B einen ersten Bereich 184 auf, in welchem das Funktionsbauteil 114 beispielsweise kraft- oder formschlüssig aufgenommen wird, und einen Freiraum 186 im Bereich der Insertionsstelle 152. Durch diesen Freiraum 186 verbleibt die Insertionsstelle 152 auch in dem in Figur 4 dargestellten Zustand, in welchem das Schutzbauteil 160 auf das Funktionsbauteil 114 und die Funktionsbaugruppe 154 aufgesetzt ist, von oben zugänglich und sichtbar, beispielsweise für eine visuelle Beobachtung oder eine Reinigung. Die Verbindung des Schutzbauteils 160 mit dem Funktionsbauteil 114 erfolgt vorzugsweise reversibel, so dass auch im Betrieb der Funktionsbaugruppe 154, beispielsweise im Messbetrieb, eine medizinische Funktion der Funktionsbaugruppe 154 unbeeinflusst von einer Abnahme des Schutzbauteils 160 ist. Dies kann durch verschiedene Ausgestaltungen des Schutzbauteils 160 unterstützt werden. Beispielsweise kann - wie in Figur 3B erkennbar - die Öffnung 182 im Bereich des insertierbaren Elements 116 eine Abschrägung 188 in der Nähe der Insertionsstelle 152 oder oberhalb des Insertionsbereichs 156 aufweisen. Weiterhin kann das Funktionsbauteil 114, wie oben erläutert, eine Schnittstelle 126 aufweisen (siehe Figur 5B), welche beispielsweise über ein Kabel angebunden sein kann. Das Schutzbauteil 160 kann im Bereich dieser Schnittstelle 126 beispielsweise eine Kabeldurchführung 190 aufweisen, beispielsweise in Form einer nach unten, zur Hautoberfläche 148 hin geöffneten Nut, durch welche ein Kabel oder eine andere Anbindung der Schnittstelle 126 hindurchgeführt werden kann, so dass das Schutzbauteil 160 nach Belieben montierbar oder abnehmbar ist, ohne die Schnittstelle 126 und deren Kontaktierung zu beeinflussen und somit ohne die Funktion der Funktionsbaugruppe 154 unterbrechen zu müssen. Das Schutzbauteil 160 kann beispielsweise vollständig inert ausgestaltet sein und rein mechanische Schutzfunktionen aufweisen. Optional kann jedoch, was in Figur 4 angedeutet ist, mindestens eine Schnittstelle 192 zwischen dem Schutzbauteil 160 und dem Funktionsbauteil 114 existieren, welche beim Verbinden dieser Bauteile geschlossen wird, so dass beispielsweise auch eine elektrische Verbindung zwischen diesen Bauteilen hergestellt werden kann. Auf diese Weise können auch eine oder mehrere Komponenten der medizinischen Vorrichtung 110, welche eine Funktion aufweisen, die über eine rein mechanische Schutzfunktion hinausgeht, in dem Schutzbauteil 160 integriert sein, beispielsweise Komponenten für eine Medikationsfunktion. Auch mindestens ein weiteres insertierbares Element 116 kann vorgesehen sein, welches beispielsweise mit dem Schutzbauteil 160 verbunden werden kann, beispielsweise ein Katheter einer Medikationsvorrichtung.

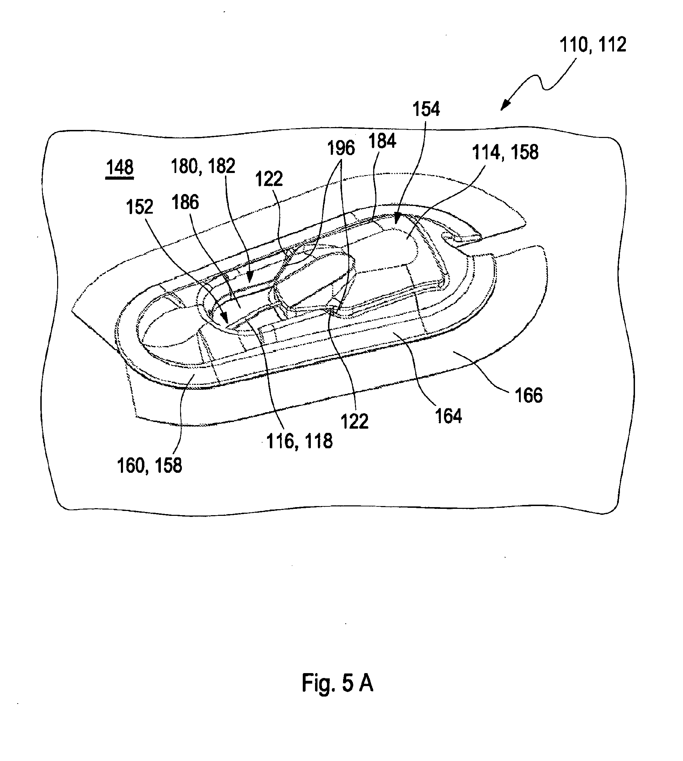

Wie in Figur 4 erkennbar ist, wird die Funktionsbaugruppe 154 durch das Aufsetzen des Schutzbauteils 160 mechanisch geschützt, wobei das Schutzbauteil 160 das Funktionsbauteil 114 und vorzugsweise auch den Insertionsbereich 156 abdeckt, bzw. ringförmig (in diesem Fall beispielsweise oval) umgibt. Nach dem Aufsetzen kann durch Zug an den Abziehlaschen 178 der Liner 174 von der selbstklebenden Oberfläche 172 des Schutzbauteils 160 entfernt werden. Damit klebt auch die Auflagefläche 170 des Schutzbauteils 160 auf der Hautoberfläche 148 und umgibt die Auflagefläche 132 des Funktionsbauteils 114, welche ebenfalls auf der Hautoberfläche 148 klebt. Dieser Zustand ist in den Figuren 5A und 5B dargestellt. Dabei zeigt Figur 5A eine Draufsicht auf die Anordnung gemäß Figur 4 mit abgezogenem Liner 174, und Figur 5B zeigt eine Schnittdarstellung durch die fertige, auf die Hautoberfläche 148 aufgebrachte medizinische Vorrichtung 110. Hierbei ist, wie oben dargestellt, auch erkennbar, dass das Funktionsbauteil 114 eine oder mehrere Funktionskomponenten 124 umschließen kann, welche beispielsweise in das Funktionsbauteil 114 integriert oder innerhalb eines Innenraums 194 des Funktionsbauteils 114 aufgenommen sein können. Aus dieser Schnittdarstellung gemäß Figur 5B ist auch erkennbar, dass der Insertionsbereich 156 vorzugsweise vollständig von dem Gehäuse 158 überdeckt ist, mit Ausnahme des Freiraums 186 oberhalb der Insertionsstelle 152. Die Auflagefläche 170 des Schutzbauteils 160 umgibt die Auflagefläche 132 des Funktionsbauteils 114 vorzugsweise vollständig. Die Öffnung 182 der Aufnahme 180 ist, wie in Figur 5A erkennbar, vorzugsweise derart dimensioniert, dass der erste Bereich 184 in seiner Form an die äußere Kontur des Funktionsbauteils 114 angepasst ist, so dass sich dieses innerhalb der Öffnung 182 nicht verdrehen kann oder nicht innerhalb dieser Öffnung 182 verrutschen kann. Zu diesem Zweck können beispielsweise eine oder mehrere Schultern 196 vorgesehen sein, welche mit den abgeschrägten Kanten 122 des Funktionsbauteils 114 zusammenwirken, so dass dieses nicht in den Freiraum 186 hinein verrutschen kann, sondern in seiner Waage relativ zur Insertionsstelle 152 fixiert ist. Somit ist weder eine Verdrehung noch eine Verschiebung innerhalb der Öffnung 182 in nennenswertem Maße möglich. Sowohl das Funktionsbauteil 114 als auch das Schutzbauteil 160 können aus geeigneten Materialien hergestellt sein, vorzugsweise aus Materialien, welche flexible oder verformbare Eigenschaften aufweisen. Dabei kann das Schutzbauteil 160 grundsätzlich aus einem weicheren Material hergestellt sein als das Funktionsbauteil 114. Beispielsweise kommen Polyurethane und/oder Silikone als geeignete Materialien in Betracht.

Weiterhin ist in Figur 5B erkennbar, dass das Gehäuse 158 optional weitere Bauelemente umfassen kann. So ist in Figur 5B angedeutet, dass weiterhin eine optionale Schutzabdeckung 198 über die gesamte Baugruppe aufbringbar ist, welche ebenfalls Bestandteil des Gehäuses 158 sein kann und welche beispielsweise als selbstklebendes transparentes Pflaster 200 ausgestaltet sein kann. Das transparente Pflaster 200 kann lateral über das Schutzbauteil 160 hinausragen und beispielsweise ebenfalls teilweise auf der Hautoberfläche 148 aufgeklebt sein. Die optionale Transparenz des transparenten Pflasters 200 ermöglicht optional weiterhin eine Beobachtung der Insertionsstelle 152 durch das transparente Pflaster 200 und durch den Freiraum 186 hindurch. Das transparente Pflaster 200 kann, beispielsweise gemeinsam mit dem Schutzbauteil 160, ausgetauscht werden, ohne die Funktion der Funktionsbaugruppe 154 zu stören, nach Belieben entfernt und beispielsweise ausgetauscht werden. Auf diese Weise bilden alle Komponenten der medizinischen Vorrichtung 110, welche oberhalb der Hautoberfläche 148 angeordnet sind, eine Basis 202 der medizinischen Vorrichtung 110, welche mit dem mindestens einen insertierbaren Element 116 zusammenwirkt. Beispielsweise kann es sich hierbei um eine Sensorbasis handeln. Diese Basis 202 umfasst das Gehäuse 158, welches mehrteilig ausgestaltet ist und beispielsweise neben dem Funktionsbauteil 114 das Schutzbauteil 160 umfasst, die nacheinander aufgebracht werden können. Auf diese Weise ist ein optimaler Schutz des insertierten insertierbaren Elements 116 gewährleistet.

**Bezugszeichenliste**

| | | | |
|---|---|---|---|
| 110 | Medizinische Vorrichtung zur Ausübung mindestens einer Funktion | 168 | Unterseite |
| | | 170 | Auflagefläche des Schutzbauteils |
| | | 172 | selbstklebende Oberfläche des Schutzbauteils |
| 112 | Sensorvorrichtung | | |
| 114 | Funktionsbauteil | 174 | Liner |
| 116 | insertierbares Element | 176 | Kanten |
| 118 | insertierbarer Sensor | 178 | Abziehlaschen |
| 120 | Stirnseite | 180 | Aufnahme |
| 122 | abgeschrägte Kanten | 182 | Öffnung |
| 124 | Funktionskomponente | 184 | erster Bereich |
| 126 | Schnittstelle | 186 | Freiraum |
| 128 | Oberseite des Funktionsbauteils | 188 | Abschrägung |
| 130 | Unterseite des Funktionsbauteils | 190 | Kabeldurchführung |
| 132 | Auflagefläche des Funktionsbauteils | 192 | Schnittstelle |
| | | 194 | Innenraum |
| 134 | selbstklebende Oberfläche des Funktionsbauteils | 196 | Schultern |
| | | 198 | Schutzabdeckung |
| 136 | Pflaster | 200 | transparentes Pflaster |
| 138 | Liner | 202 | Basis |
| 140 | Kante | | |
| 142 | Abziehlasche | | |
| 144 | Abschrägung | | |
| 146 | Vertiefung | | |
| 148 | Hautoberfläche | | |
| 150 | Körpergewebe | | |
| 152 | Insertionsstelle | | |
| 154 | Funktionsbaugruppe | | |
| 156 | Insertionsbereich | | |
| 158 | Gehäuse | | |
| 160 | Schutzbauteil | | |
| 162 | Oberseite des Schutzbauteils | | |
| 164 | Schutzgehäuse | | |
| 166 | Pflaster | | |

## Patentansprüche

1. Medizinische Vorrichtung (110) zur Ausübung mindestens einer medizinischen Funktion, umfassend mindestens ein zumindest teilweise in ein Körpergewebe (150) eines Benutzers insertierbares Element (116), weiterhin umfassend mindestens ein auf einer Hautoberfläche (148) des Benutzers aufbringbares Gehäuse (158), wobei das Gehäuse (158) mehrteilig ausgestaltet ist und mindestens ein Funktionsbauteil (114) umfasst, wobei das Funktionsbauteil (114) mit dem insertierbaren Element (116) verbindbar ist, wobei das Gehäuse (158) weiterhin mindestens ein Schutzbauteil (160) aufweist, wobei das Schutzbauteil (160) eingerichtet ist, um das Funktionsbauteil (114) zumindest teilweise zu umschließen, wobei das Schutzbauteil (160) mit dem Funktionsbauteil (114) verbindbar ist, insbesondere nach einer Insertion des insertierbaren Elements (116), **dadurch gekennzeichnet dass** das Funktionsbauteil (114) und das Schutzbauteil (160) jeweils mindestens eine der Hautoberfläche (148) zuweisende selbstklebende Oberfläche (134, 172) aufweisen.

2. Medizinische Vorrichtung (110) nach dem vorhergehenden Anspruch, wobei das Schutzbauteil (160) das Funktionsbauteil (114) derart umschließt, dass mindestens eine Insertionsstelle (152), an welcher das insertierbare Element (116) die Hautoberfläche (148) des Benutzers durchdringt, zugänglich ist.

3. Medizinische Vorrichtung (110) nach dem vorhergehenden Anspruch, wobei die Insertionsstelle (152) in einer Richtung parallel zur Hautoberfläche (148) vollständig von dem Gehäuse (158) umgeben ist.

4. Medizinische Vorrichtung (110) nach einem der vorhergehenden Ansprüche, wobei ein äußerer Rand des Schutzbauteils (160) eine Projektion des Funktionsbauteils (114) und des insertierbaren Elements (116) auf die Hautoberfläche (148) des Benutzers vollständig umschließt.

5. Medizinische Vorrichtung (110) nach einem der vorhergehenden Ansprüche, wobei die Insertionsstelle (152) im Wesentlichen mittig zu einer Auflagefläche (132, 170) des Gehäuses (158) auf der Hautoberfläche (148) angeordnet ist.

6. Medizinische Vorrichtung (110) nach einem der vorhergehenden Ansprüche, wobei das insertierbare Element (116) ausgewählt ist aus: einem Sensor (118) zum Nachweis mindestens eines Analyten in einer Körperflüssigkeit; einem Bestandteil einer Medikationsvorrichtung, insbesondere einer Insulinpumpe.

7. Medizinische Vorrichtung (110) nach einem der vorhergehenden Ansprüche, wobei mindestens eine Funktion des Funktionsbauteils (114) unabhängig von dem Vorhandensein des Schutzbauteils (160) ausübbar ist, insbesondere eine medizinische Funktion, welche von dem Funktionsbauteil (114) in Zusammenwirkung mit dem insertierbaren Element (116) ausübbar ist, vorzugsweise eine Nachweisfunktion und/oder eine Medikationsfunktion.

8. Medizinische Vorrichtung (110) nach einem der vorhergehenden Ansprüche, wobei das Gehäuse (158) zumindest teilweise flexibel ausgestaltet ist.

9. Medizinische Vorrichtung (110) nach einem der vorhergehenden Ansprüche, wobei das insertierbare Element (116) an einer Seite (120) des Funktionsbauteils (114) mit dem Funktionsbauteil (114) verbunden ist, wobei das Funktionsbauteil (114) mindestens eine dieser Seite (120) zuweisende, schräg zur Hautoberfläche (148) in Richtung dieser Seite verlaufende Vertiefung (146) zur Erleichterung einer Insertion aufweist.

10. Medizinische Vorrichtung (110) nach einem der vorhergehenden Ansprüche, wobei das Gehäuse (158) weiterhin mindestens eine Schutzabdeckung (198) aufweist, wobei die Schutzabdeckung (198) über das Schutzbauteil (160) und das Funktionsbauteil (114) aufbringbar ist und das Schutzbauteil (160) und das Funktionsbauteil (114) zumindest teilweise auf einer der Hautoberfläche (148) abgewandten Seite überdeckt.

11. Medizinische Vorrichtung (110) nach einem der vorhergehenden Ansprüche, wobei das Funktionsbauteil (114) mindestens eine mit dem insertierbaren Element (116) verbindbare elektronische und/oder elektromechanische und/oder fluidische Funktionskomponente (124) umschließt.

12. Medizinische Vorrichtung (110) nach einem der vorhergehenden Ansprüche, wobei das Schutzbauteil (160) mindestens eine elektronische und/oder elektromechanische und/oder fluidische Funktionskomponente umschließt.

13. Schutzbauteil (160) zum Einsatz in einer medizinischen Vorrichtung (110) nach einem der vorhergehenden Ansprüche, wobei das Schutzbauteil (160) mindestens eine Auflagefläche (170) zur Auflage auf eine Hautoberfläche (148) des Benutzers aufweist, wobei das Schutzbauteil (160) mit dem Funktionsbauteil (114) verbindbar ist, wobei das Schutzbauteil (160) mindestens eine Aufnahme (180) mit mindestens einer Öffnung (182) aufweist, wobei die Aufnahme (180) derart eingerichtet ist, dass das Funktionsbauteil (114) in die Aufnahme (180) einbringbar ist, wobei das Schutzbauteil (160) das Funktionsbauteil (114) in verbundenem Zustand zumindest teilweise umschließt.

14. Verfahren zum Schutz mindestens eines auf eine Hautoberfläche (148) eines Benutzers aufgebrachten Funktionsbauteils (114) einer medizinischen Vorrichtung (110) und mindestens eines mit dem Funktionsbauteil (114) verbundenen, in ein Körpergewebe (150) des Benutzers insertierbaren Elements (116), wobei eine medizinische Vorrichtung (110) nach einem der vorhergehenden, eine medizinische Vorrichtung (110) betreffenden Ansprüche verwendet wird, wobei mindestens ein Schutzbauteil (160) mit dem Funktionsbauteil (114) verbunden wird, wobei das Schutzbauteil (160) das Funktionsbauteil (114) zumindest teilweise umschließt.

## Claims

1. Medical device (110) for carrying out at least one medical function, comprising at least one element (116) that can be at least partially inserted into a body tissue (150) of a user and further comprising at least one housing (158) that can be placed on a skin surface (148) of the user, wherein the housing (158) has a multipart design and comprises at least one functional component (114), wherein the functional component (114) can be connected to the insertable element (116), wherein the housing (158) further comprises at least one protective component (160), wherein the protective component (160) is designed to at least partially enclose the functional component (114), wherein the protective component (160) can be connected to the functional component (114), particularly after insertion of the insertable element (116), **characterized in that** the functional component (114) and the protective component (160) each have at least one self-adhesive surface (134, 172) facing toward the skin surface (148).

2. Medical device (110) according to the preceding claim, wherein the protective component (160) encloses the functional component (114) in such a way that at least one insertion site (152), where the insertable element (116) passes through the skin surface (148) of the user, is accessible.

3. Medical device (110) according to the preceding claim, wherein the insertion site (152), in a direction parallel to the skin surface (148), is completely surrounded by the housing (158).

4. Medical device (110) according to one of the preceding claims, wherein an outer edge of the protective component (160) completely encloses a projection of the functional component (114) and of the insertable element (116) onto the skin surface (148) of the user.

5. Medical device (110) according to one of the preceding claims, wherein the insertion site (152) is arranged substantially centrally with respect to a contact face (132, 170) of the housing (158) on the skin surface (148).

6. Medical device (110) according to one of the preceding claims, wherein the insertable element (116) is chosen from: a sensor (118) for detection of at least one analyte in a body fluid; a constituent part of a medication device, in particular an insulin pump.

7. Medical device (110) according to one of the preceding claims, wherein at least one function of the functional component (114) can be carried out independently of the presence of the protective component (160), in particular a medical function that can be carried out by the functional component (114) in cooperation with the insertable element (116), preferably a detection function and/or a medication function.

8. Medical device (110) according to one of the preceding claims, wherein the housing (158) is designed to be at least partially flexible.

9. Medical device (110) according to one of the preceding claims, wherein the insertable element (116) is connected to the functional component (114) on one side (120) of the functional component (114), wherein the functional component (114), in order to facilitate an insertion, has at least one depression (146) facing toward this side (120) and extending obliquely with respect to the skin surface (148) in the direction of this side.

10. Medical device (110) according to one of the preceding claims, wherein the housing (158) further has at least one protective covering (198), wherein the protective covering (198) can be placed over the protective component (160) and the functional component (114) and covers the protective component (160) and the functional component (114) at least partially on a side directed away from the skin surface (148).

11. Medical device (110) according to one of the preceding claims, wherein the functional component (114) encloses at least one electronic and/or electromechanical and/or fluidic functional module (124) that can be connected to the insertable element (116).

12. Medical device (110) according to one of the preceding claims, wherein the protective component (160) encloses at least one electronic and/or electromechanical and/or fluidic functional module.

13. Protective component (160) for use in a medical device (110) according to one of the preceding claims, wherein the protective component (160) has at least one contact face (170) for placing on a skin surface (148) of the user, wherein the protective component (160) can be connected to the functional component (114), wherein the protective component (160) has at least one seat (180) with at least one opening (182), wherein the seat (180) is designed in such a way that the functional component (114) can be introduced into the seat (180), wherein the protective component (160) at least partially encloses the functional component (114) in the connected state.

14. Method for protecting at least one functional component (114) of a medical device (110) that is placed onto a skin surface (148) of a user and for protecting at least one element (116) that can be connected to the functional component (114) and can be inserted into a body tissue (150) of the user, in particular using a medical device (110) according to one of the preceding claims relating to a medical device (110), wherein at least one protective component (160) is connected to the functional component (114), wherein the protective component (160) at least partially encloses the functional component (114).

## Revendications

1. Dispositif médical (110) destiné à exercer au moins une fonction médicale, comprenant au moins un élément (116) pouvant être inséré au moins en partie dans un tissu corporel (150) d'un utilisateur, comprenant en outre au moins un boîtier (158) pouvant être appliqué sur une surface de la peau (148) de l'utilisateur, le boîtier (158) étant réalisé en plusieurs parties et comprenant au moins un composant fonctionnel (114), le composant fonctionnel (114) pouvant être connecté à l'élément insérable (116), le boîtier (158) présentant en outre au moins un composant de protection (160), le composant de protection (160) étant prévu pour entourer au moins en partie le composant fonctionnel (114), le composant de protection (160) pouvant être connecté au composant fonctionnel (114), notamment après une insertion de l'élément insérable (116), **caractérisé en ce que** le composant fonctionnel (114) et le composant de protection (160) présentent chacun au moins une surface autocollante (134, 172) tournée vers la surface de la peau (148).

2. Dispositif médical (110) selon la revendication précédente, dans lequel le composant de protection (160) entoure le composant fonctionnel (114) de telle sorte que l'on puisse accéder à au moins une zone d'insertion (152) au niveau de laquelle l'élément insérable (116) traverse la surface de la peau (148) de l'utilisateur.

3. Dispositif médical (110) selon la revendication précédente, dans lequel la zone d'insertion (152), dans une direction parallèle à la surface de la peau (148), est complètement entourée par le boîtier (158).

4. Dispositif médical (110) selon l'une quelconque des revendications précédentes, dans lequel un bord extérieur du composant de protection (160) entoure complètement une partie saillante du composant fonctionnel (114) et de l'élément insérable (116) sur la surface de la peau (148) de l'utilisateur.

5. Dispositif médical (110) selon l'une quelconque des revendications précédentes, dans lequel la zone d'insertion (152) est disposée essentiellement centralement par rapport à une surface d'appui (132, 170) du boîtier (158) sur la surface de la peau (148).

6. Dispositif médical (110) selon l'une quelconque des revendications précédentes, dans lequel l'élément insérable (116) est choisi parmi les éléments suivants : un capteur (118) pour mettre en évidence au moins un analyte dans un liquide corporel ; un constituant d'un dispositif de médication, en particulier d'une pompe à insuline.

7. Dispositif médical (110) selon l'une quelconque des revendications précédentes, dans lequel au moins une fonction du composant fonctionnel (114) peut être exercée indépendamment de la présence du composant de protection (160), en particulier une fonction médicale, qui peut être exercée par le composant fonctionnel (114) coopérant avec l'élément insérable (116), de préférence une fonction de mise en évidence et/ou une fonction de médication.

8. Dispositif médical (110) selon l'une quelconque des revendications précédentes, dans lequel le boîtier (158) est configuré de manière au moins partiellement flexible.

9. Dispositif médical (110) selon l'une quelconque des revendications précédentes, dans lequel l'élément insérable (116) est connecté au composant fonctionnel (114) au niveau d'un côté (120) du composant fonctionnel (114), le composant fonctionnel (114) présentant au moins un renfoncement (146) tourné vers ce côté (120), s'étendant obliquement par rapport à la surface de la peau (148) dans la direction de ce côté, pour faciliter une insertion.

10. Dispositif médical (110) selon l'une quelconque des revendications précédentes, dans lequel le boîtier (158) présente en outre au moins un recouvrement de protection (198), le recouvrement de protection (198) pouvant être appliqué pardessus le composant de protection (160) et le composant fonctionnel (114) et recouvrant au moins en partie le composant de protection (160) et le composant fonctionnel (114) sur un côté opposé à la surface de la peau (148).

11. Dispositif médical (110) selon l'une quelconque des revendications précédentes, dans lequel le composant fonctionnel (114) entoure au moins un constituant fonctionnel (124) électronique et/ou électromécanique et/ou fluidique pouvant être connecté à l'élément insérable (116).

12. Dispositif médical (110) selon l'une quelconque des revendications précédentes, dans lequel le composant de protection (160) entoure au moins un constituant fonctionnel électronique et/ou électromécanique et/ou fluidique.

13. Composant de protection (160) pour l'utilisation dans un dispositif médical (110) selon l'une quelconque des revendications précédentes, dans lequel le composant de protection (160) présente au moins une surface d'appui (170) destinée à l'appui sur une surface de la peau (148) de l'utilisateur, le composant de protection (160) pouvant être connecté au composant fonctionnel (114), le composant de protection (160) présentant au moins un logement (180) avec au moins une ouverture (182), le logement (180) étant réalisé de telle sorte que le composant fonctionnel (114) puisse être introduit dans le logement (180), le composant de protection (160) entourant au moins en partie le composant fonctionnel (114) dans l'état connecté.

14. Procédé pour protéger au moins un composant fonctionnel (114) d'un dispositif médical (110) appliqué sur une surface de la peau (148) d'un utilisateur et au moins un élément (116) connecté au composant fonctionnel (114), pouvant être inséré dans un tissu corporel (150) de l'utilisateur, un dispositif médical (110) selon l'une quelconque des revendications précédentes concernant un dispositif médical (110) étant utilisé, au moins un composant de protection (160) étant connecté au composant fonctionnel (114), le composant de protection (160) entourant au moins en partie le composant fonctionnel (114).
